# FASCICULE DE BREVET EUROPEEN

(11) **EP 0 887 350 B1**
(45) Date de publication et mention de la délivrance du brevet: **22.08.2001**
(21) Numéro de dépôt: 98401541.2
(22) Date de dépôt: 23.06.1998
(51) Int. Cl.: C07D 491/052, A61K 31/435, A61K 31/40

(54) **Nouveaux dérivés chroméniques, leur procédé de préparation et les compositions pharmaceutiques qui les contiennent**
Chromenderivate, Verfahren zu ihrer Herstellung und diese enthaltende pharmazeutische Zubereitungen
Chromene derivatives, process for their preparation and pharmaceutical compositions containing them

(30) Priorité: 24.06.1997 FR 9707839
(43) Date de publication de la demande: 30.12.1998
(73) Titulaire: ADIR ET COMPAGNIE, 92415 Courbevoie Cédex (FR)
(72) Inventeur: Lavielle, Gilbert, 78170 La Celle Saint Cloud (FR); Dubuffet, Thierry, 94550 Chevilly-la-Rue (FR); Hautefaye, Patrick, 77170 Servon Brie Comte Robert (FR); Lejeune, Françoise, 92210 Saint Cloud (FR); Millan, Mark, 78230 Le Pecq (FR)

(56) Documents cités:
- EP-A- 0 691 342

## Description

La présente invention concerne de nouveaux dérivés chroméniques, leur procédé de préparation, et les compositions pharmaceutiques qui les contiennent.

Les composés de la présente invention, outre le fait qu'ils soient nouveaux, présentent des propriétés particulièrement intéressantes, en se fixant de manière sélective aux récepteurs D₃ par rapport aux récepteurs D₂.

La découverte de ces récepteurs dopaminergiques D₃ (P. Sokoloff et al., Nature, 1990, 347, 147), leur forte concentration au niveau du système limbique, et leur faible densité au niveau des cellules lacotrophes et dans le système nigrostrié, en font une cible de choix pour l'obtention d'antipsychotiques dépourvus d'effets sur la sécrétion de prolactine et moins susceptible de provoquer des syndromes de type extra pyramidaux. Il est en effet bien établi que les voies dopaminergiques projetant vers le système limbique et le cortex jouent un rôle déterminant dans le contrôle de l'humeur, et dans l'étiologie et le traitement des maladies psychiatriques telles que la schizophrénie, la dépression, l'anxiété, l'agression et les autres troubles des impulsions (M.J. Millan et al., Drug News & Perspectives, 1992, 5, 397-406 ; A.Y. Deutch et al., Schizophrenia, 1991, *4*, 121-156 ; H.Y. Meltzen et al., Pharmacol. Rev., 1991, 43, 587-604).

Les composés de l'art antérieur les plus proches de ceux qui font l'objet de cette demande (EP 691342 J. Med. Chem., 1989, 32, 720-7) ont été décrits pour leurs propriétés dopaminergiques ou serotoninergiques. Les composés de la présente demande se caractérisent par la présence de substituants attracteurs de type nitrile ou carboxamides qui permettent, de façon surprenante, d'exalter en terme de puissance et de sélectivité les propriétés dopaminergiques D₃. Cette sélectivité confère aux produits de l'invention un intérêt tout particulier pour leur utilisation comme médicaments agissant au niveau du système dopaminergique, dépourvus des effets indésirables des ligands D₂. A la lumière de résultats parus dans la littérature, ils sont susceptibles d'être utilisés dans le traitement des maladies impulsives (par exemple celles provoquées par l'abus de drogue B. Caine, Science, 1993, 260, 1814), de l'agressivité (J.W. Tidey, Behavional Pharm., 1992, 3, 553), de la maladie de Parkinson (J. Carlson, Neur. Transm., 1993, *94*, 11), des psychoses, des troubles de la mémoire (P. Sokoloff et al., Nature, 1990, *347*, 147), de l'anxiété et de la dépression (P. Willner, Clinical Neuropharm., 1985, *18*, suppl. 1, 549-56).

Plus spécifiquement, la présente invention concerne les composés de formule (I) : 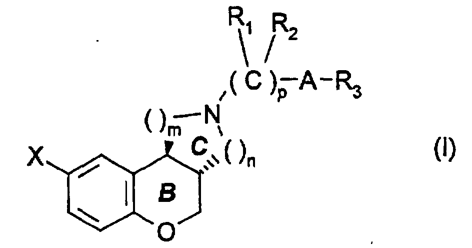 dans laquelle :
- ( ) représente un groupement -CH₂-,
- m est un entier tel que 0 ≤ m ≤ 3,
- n est un entier tel que 0 ≤ n ≤ 3 et 2 ≤ m+n ≤ 3,
- p est un entier tel que 1 ≤ p ≤ 6,
- la jonction entre les cycles *B* et *C* est de configuration trans,
- X représente un groupement cyano, ou un groupement -CO-NR₄R₅, R₄ et R₅ étant choisis parmi hydrogène, alkyle (C₁-C₆) linéaire ou ramifié, cycloalkyle (C₃-C₇), aryle éventuellement substitué,
- A représente une liaison σ ou un groupement choisi parmi -NR-CO-, -CO-NR-, -NR-SO₂, -SO₂-NR, dans lesquels R représente un atome d'hydrogène ou un groupement alkyle (C₁-C₆) linéaire ou ramifié,
- R₁ et R₂ représentent indépendamment un atome d'hydrogène ou un groupement alkyle (C₁-C₆) linéaire ou ramifié,
- R₃ représente :
   - un atome d'hydrogène, un groupement phényle, naphtyle ou hétéroaryle, chacun de ces groupements étant éventuellement substitué par un ou plusieurs atomes d'halogènes, groupements alkyle (C₁-C₆) linéaire ou ramifié, alkoxy (C₁-C₆) linéaire ou ramifié, hydroxy, cyano, amino, nitro, carboxy, perhalogénoalkyle (C₁-C₆) linéaire ou ramifié, sulfo, acylamino, alkyl (C₁-C₆) sulfonyle linéaire ou ramifié ou alkyle (C₁-C₆) sulfonylamino linéaire ou ramifié,
   - un groupement aryle ou hétéroaryle substitué par un groupement A'-Cy dans lequel A' représente une liaison σ ou un groupement alkylène (C₁-C₆) linéaire ou ramifié (dans lequel un atome de carbone peut être éventuellement remplacé par un atome d'oxygène ou de soufre), alkénylène (C₁-C₆) linéaire ou ramifié (dans lequel un atome de carbone peut être éventuellement remplacé par un atome d'oxygène ou de soufre), un groupement -NR-CO-, -CO-NR, -NR-SO₂-, SO₂-NR, dans lesquels R représente un atome d'hydrogène ou un groupement alkyle (C₁-C₆) linéaire ou ramifié, et Cy représente un groupement aryle éventuellement substitué, ou hétéroaryle éventuellement substitué,
   - un groupement 2-indolinone-5-yl,
   - ou bien un groupement aryloxy ou arylthio, à la condition que dans ce cas A représente une liaison σ,
étant entendu que :
- lorsque n est égal à 0, alors m est différent de 2,
- lorsque n est égal à 1, R₁ et R₂ représentent un hydrogène, A représente une liaison σ et p est égal à 1, alors R₃ est différent de phényle et pyridyle,
- lorsque n est égal à 1, R₁ et R₂ représentent un hydrogène, A représente une liaison σ, alors R₃ est différent d'un atome d'hydrogène,
- lorsque n est égal à 1, R₁ et R₂ représentent un hydrogène, A représente un groupement -NH-CO-, alors R₃ est différent d'un atome d'hydrogène, d'un groupement phényle, naphtyle, hétérocyclique choisi parmi thiényle, furyle, pyrrolyle, pyridyle, chacun de ces groupements étant éventuellement substitué par un ou plusieurs atomes d'halogène, ou groupements trihalogénométhyle, alkoxy ou hydroxy,
leurs isomères, énantiomères, diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, acétique, trifluoroacétique, lactique, malonique, succinique, glutamique, fumarique, maléïque, citrique, oxalique, méthane sulfonique, benzène sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement aryle, on entend un groupement phényle ou naphtyle.

Par groupement hétéroaryle, on entend un groupement mono ou bi-cyclique aromatique comportant de 5 à 13 chaînons, et un à quatre hétéroaromes choisis parmi azote, oxygène ou soufre, par exemple un groupement furyle, pyridyle ou thiényle.

Le terme éventuellement substitué affecté aux expressions aryle et hétéroaryle signifie que ces groupements sont éventuellement substitué par un ou plusieurs atomes d'halogène, ou groupements alkyle (C₁-C₆) linéaire ou ramifié, hydroxy, alkoxy (C₁-C₆) linéaire ou ramifié, perhalogénoalkyle (C₁-C₆) linéaire ou ramifié, cyano, nitro, amino, sulfo, acyle (C₁-C₆) linéaire ou ramifié, acylamino, alkyl (C₁-C₆) sulfonyle linéaire ou ramifié, ou alkyl (C₁-C₆)sulfonylamino linéaire ou ramifié.

Le terme acyle, seul ou dans l'expression "acylamino", représente un groupement alkylcarbonyle (C₁-C₆) linéaire ou ramifié, ou cycloalkylcarbonyle (C₃-C₈).

Préférentiellement l'invention concerne les dérivés de formule (I) pour lesquels m et n sont tous les deux égaux à 1.

D'autres composés préférés de l'invention sont ceux pour lesquels m est égal à 3 tandis que n vaut 0.

Dans les composés de formule (I) X représente préférentiellement un groupement cyano.

Dans les composés préférés de l'invention, A représente une liaison σ ou un groupement NR-CO- ou NR-SO₂, R étant préférentiellement un atome d'hydrogène.

Dans les composés de formule (I), R₁ et R₂ représentent plus spécifiquement chacun un atome d'hydrogène.

Les groupements R₃ préférés de l'invention sont les groupements phényle éventuellement substitué, ou biphényle éventuellement substitué.

Un autre groupement R₃ préféré est le groupement aryle (plus particulièrement phényle) substitué par un groupement A'-Cy dans lequel A' représente préférentiellement un groupement NR-CO ou NR-SO₂ (R étant plus particulièrement un atome d'hydrogène), et Cy représente préférentiellement un groupement aryle éventuellement substitué.

Un autre groupement R₃ préféré est le groupement 2-indolinone-5-yle.

L'invention concerne plus préférentiellement les composés de formule (I) pour lesquels X représente un groupement cyano, m et n sont tous les deux égaux à 1, R₁ et R₂ représentent chacun un atome d'hydrogène, et p vaut 4 tandis que A représente un groupement NHCO et R₃ un groupement phényle éventuellement substitué ou biphényle éventuellement substitué, ou bien p vaut 1 ou 2 tandis que A représente une liaison σ et R₃ un groupement phényle éventuellement substitué ou biphényle éventuellement substitué.

L'invention s'étend également au procédé de préparation des composés de formule (I), caractérisé en ce que l'on utilise comme produit de départ un composé de formule (II) : 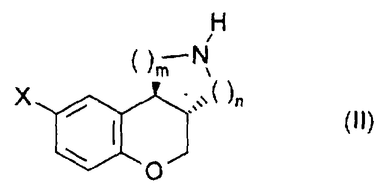 dans laquelle X, m et n ont la même signification que dans la formule (I), que l'on traite,
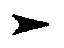 soit par un composé de formule (III) : 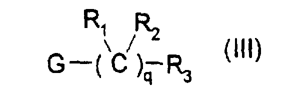 dans laquelle G représente un atome d'halogène ou un groupement CHO, q est un entier tel que 0 ≤ q ≤ 6, et R₁, R₂ et R₃ sont tels que définis dans la formule (I),
   pour conduire, après réduction lorsque G représente un groupement CHO, à un dérivé de formule (l/a) : 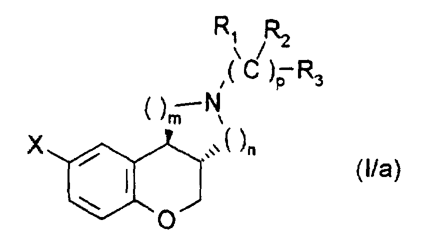 cas particulier des dérivés de formule (I) pour lesquels X, R₁, R₂, R₃, m, n et p ont la même signification que dans la formule (I),
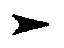 soit par un composé de formule (IV) : 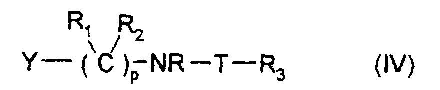 dans laquelle Y représente un atome d'halogène, R, R₁, R₂, R₃ et p sont tels que définis dans la formule (I), et T représente un groupement CO ou SO₂,
   pour conduire à un dérivé de formule (l/b) : 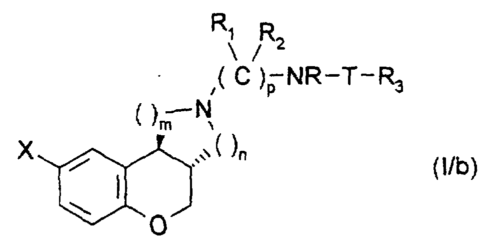 cas particulier des dérivés de formule (I), pour lesquels X, R, R₁, R₂, R₃, m, n et p ont la même signification que dans la formule (I), et T est tel que défini précédemment,
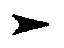 soit par un composé de formule (V) : 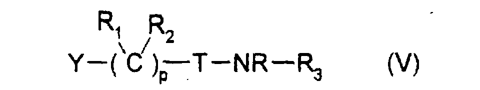 dans laquelle Y représente un atome d'halogène et R, R₁, R₂, R₃ et p sont tels que définis dans la formule (I), et T représente un groupement CO ou SO₂,
   pour conduire à un dérivé de formule (l/c) : 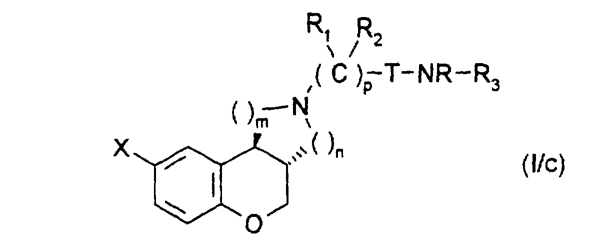 cas particulier des dérivés de formule (I), pour lequel X, R, R₁, R₂, R₃, m, n et p ont la même signification que dans la formule (I), et T est tel que défini précédemment,
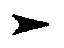 soit par un composé de formule (III') : 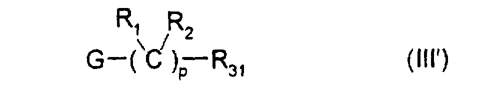 dans laquelle G représente un atome d'halogène ou un groupement CHO, p, R₁, R₂, sont tels que définis dans la formule (I), et R₃₁ représente un groupement aryle ou hétéroaryle substitué par un atome d'halogène ou groupement carboxy, nitro ou sulfo,
   pour conduire à un dérivé de formule (l'/a) : 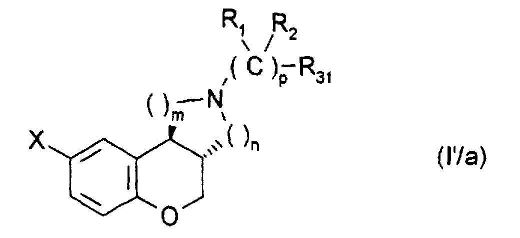 cas particulier des dérivés de formule (I/a), pour lesquels X, R₁, R₂, R₃₁, m, n et p sont tels que définis précédemment, que l'on traite :
   ∗ lorsque R₃₁ représente un groupement aryle ou hétéroaryle substitué par un atome d'halogène, par un dérivé vinylique, un dérivé de l'étain ou un dérivé de l'acide boronique approprié, en présence d'un catalyseur palladié,
      pour conduire au composé de formule (l/d) : 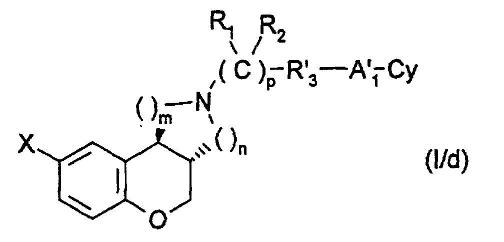 cas particulier des dérivés de formule (I), pour lequel X, R₁, R₂, R₃₁, m, n et p sont tels que définis précédemment, R'₃ représente un groupement aryle ou hétéroaryle, A'₁ représente une liaison σ ou un groupement alkylène (dans lequel un atome de carbone peut éventuellement être remplacé par un atome d'oxygène ou de soufre), alkénylène (dans lequel un atome de carbone peut éventuellement être remplacé par un atome d'oxygène ou de soufre), et Cy est tel que défini dans la formule (I),
   ∗ lorsque R₃₁ représente un groupement aryle ou hétéroaryle substitué par un groupement nitro, après réduction de ce groupement en amine, par un dérivé de formule Cl-CO-Cy ou Cl-SO₂-Cy, pour conduire au composé de formule (l/e) : 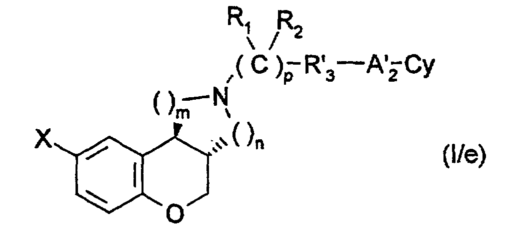 cas particulier des dérivés de formule (I), pour lequel X, R₁, R₂, m, n, p et Cy sont tels que définis dans la formule (I), R'₃ représente un groupement aryle ou hétéroaryle, et A'₂ représente un groupement -NR-CO ou -NR-SO₂-, R étant tel que défini dans la formule (I),
   ∗ lorsque R₃₁ représente un groupement aryle ou hétéroaryle substitué par un groupement sulfo ou carboxy, par un dérivé de formule HNR-Cy, pour conduire au composé de formule (l/f) : 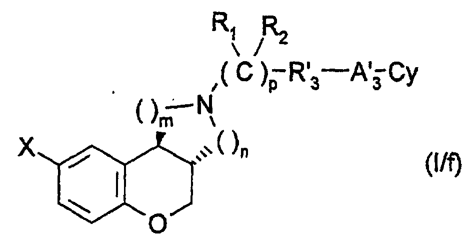 cas particulier des composés de formule (I), pour lequel X, R₁, R₂, m, n, p et Cy sont tels que définis dans la formule (I), R'₃ représente un groupement aryle ou hétéroaryle, et A'₃ représente un groupement -CO-NR-, ou -SO₂-NR-, R étant tel que défini précédemment,
   étant entendu que dans les formules précédentes ( ) représente un groupement -CH₂-,
   composés de formule (l/a) à (l/f) :
   - que l'on purifie éventuellement selon une technique classique de purification,
   - dont on sépare éventuellement les énantiomères selon une technique classique de séparation,
   - et que l'on transforme, le cas échéant, en ses sels d'addition à un acide ou à une base pharmaceutiquement acceptable,
   - étant entendu que lorsque X représente un groupement CN, il peut être transformé en groupement aminoalkyle selon des techniques classiques de la chimie organique, à tout moment de la synthèse.

Dans le but de réaliser une synthèse plus adaptée à certains produits de formule (I) que l'on souhaite obtenir, certaines variantes du procédé indiqué ci-dessus pourront être utilisées.

L'une d'entre elles consiste à utiliser comme matière première, le dérivé de formule (VI) : 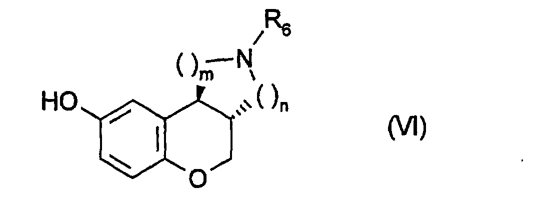 dans laquelle m et n ont la même signification que dans la formule (I) et R₆ représente un groupement alkyle (C₁-C₆) linéaire ou ramifié,
qui est traité par l'anhydride trifluorométhane sulfonique, pour conduire à un dérivé de formule (VII) : 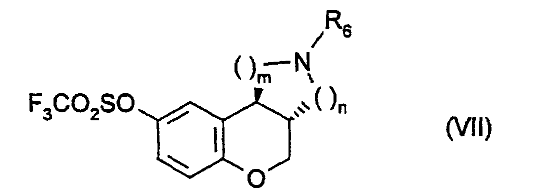 dans laquelle R₆, m et n ont la même signification que précédemment,
composé de formule (VII) qui réagit avec le cyanure de tributylétain, en présence de chlorure de lithium et d'un catalyseur au palladium (O), pour conduire au dérivé de formule (l/g) : 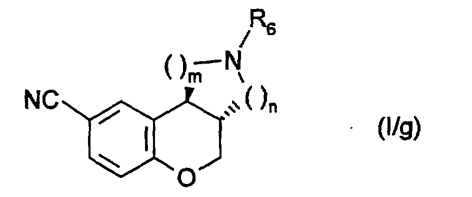 cas particulier des dérivés de formule (I) pour lesquels R₆, m et n sont tels que définis précédemment,
étant entendu que dans les formules précédentes ( ) représente un groupement -CH₂-,
composé de formule (I/g) qui peut, si nécessaire, être purifié par des méthodes classiques de purification et dont on sépare éventuellement les énantiomères par une technique classique de séparation, et qui peut être transformé, le cas échéant, en son sel d'addition à un acide ou à une base pharmaceutiquement acceptable.
La présente invention a aussi pour objet l'utilisation, pour l'obtention de compositions pharmaceutiques utiles dans le traitement des maladies nécéssitant un ligand aux récepteurs D₃, des composés de formule (VIII) : 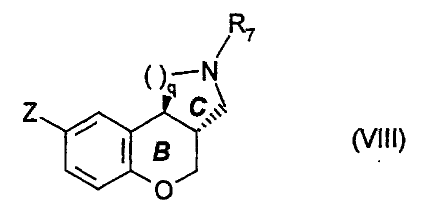 dans laquelle :
- ( ) représente un groupement -CH₂-,
- q représente 1 ou 2,
- Z représente un groupement cyano ou aminocarbonyle,
- R₇ représente un groupement alkyle (C₁-C₆) linéaire ou ramifié, benzyle, acylaminoalkyle (C₁-C₆) linéaire ou ramifié, dans lequel le groupement acyle est un groupement benzoyle, naphtylcarbonyle, thienylcarbonyle, furylcarbonyle, pyrrolylcarbonyle, pyridinylcarbonyle, chacun de ces groupements étant éventuellement substitués par un ou plusieurs atomes d'halogène, ou groupements trihalogénométhyle, alkoxy ou hydroxy,
- la jonction entre les cycles B et C est de configuration trans,
composés (VIII) décrits dans la demande EP 691 342.

L'invention s'étend également aux compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être nasale, rectale, parentérale ou orale. D'une manière générale, la posologie unitaire s'échelonne entre 1 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques usuelles.

Les préparations décrites ci-dessous conduisent à des produits de départ utilisés lors de la synthèse des composés de l'invention.

### PREPARATION A : 3-Nitrophénylacétaldéhyde

A 60 mmol de 2-(3-nitrophényl)éthanol dans 300 ml de tétrahydrofurane, sont ajoutées 90 mmol d'acide iodoxybenzoïque. Le milieu réactionnel est porté à reflux pendant 4 heures, puis refroidi et filtré. Le filtrat est concentré pour conduire au composé attendu.

### PREPARATION B : N-[4-(2-Chloroéthyl)-phényl]-acétamide

### Stade a : N-(4-Chloroacétylphényl)-acétamide

A 108 mmol de chlorure d'aluminium dans 90 ml de 1,2-dichloroéthane sont ajoutées lentement, sous atmosphère d'Argon, 23,5 mmol de chlorure de chloroacétyle et par portion 18,1 mmol d'acétanilide. Le milieu réactionnel est porté à 60°C pendant 2 heures puis refroidi par un bain de glace. Après hydrolyse lente à l'aide de glace, et filtration, le précipité obtenu est lavé à l'éther éthylique et séché. Le produit attendu ainsi obtenu est utilisé dans la suite, sans purification.

### Stade b : N-[4-(2-Chloroéthyl)-phényl]-acétamide

A 11,1 mmol de produit obtenu au stade précédent, en solution dans 10 ml d'acide trifluoroacétique, sont ajoutées lentement 22,7 mmol de triéthylsilane. L'agitation est maintenue pendant 24 heures. L'acide trifluoroacétique est ensuite éliminé et le résidu obtenu est repris par 50 ml d'acétonitrile puis lavé à l'hexane (4 fois 30 ml). Après concentration, le produit attendu est purifié par chromatographie sur gel de silice, en utilisant comme éluant un mélange dichlorométhane / acétate d'éthyle, 90/10.

### PREPARATION C : 5-(2-Chloroéthyl)-1,3-dihydro-2-oxo-indole

### Stade a : 5-Chloroacétyl-1, 3-dihydro-2-oxo-indole

Le produit attendu est obtenu selon le procédé décrit dans la Préparation B, Stade a, en remplaçant l'acétanilide par le 1,3-dihydro-2-oxo-indole.

### Stade b : 5-(2-Chloroéthyl)-1,3-dihydro-2-oxo-indole

Le produit attendu est obtenu selon le procédé décrit dans la Préparation B, Stade b, à partir du composé obtenu au Stade précédent.

### PREPARATION D : 4-Cyano-N-(4-hydroxybutyl)benzamide

A 100 mmol (16,56 g) de chlorure de 4-cyanobenzoyle dans 400 ml de dichlorométhane sont ajoutées lentement 100 mmol (9,2 ml) de 4-aminobutanol, à une température à 5°C. En maintenant la température à 5°C, 100 mmol (13,9 ml) de triéthylamine sont ajoutées. Le milieu réactionnel est agité 15 heures à température ambiante avant d'être hydrolysé. La phase organique est ensuite décantée, lavée par une solution d'acide chlorhydrique 1N. Après évaporation du solvant, le résidu obtenu est purifié par chromatographie sur gel de silice en utilisant un mélange dichlorométhane/méthanol, 97/3, comme éluant, pour conduire au produit attendu.

### PREPARATION E : 4-Phényl-N-(4-hydroxybutyl)benzamide

Le produit attendu est obtenu selon le procédé décrit dans la préparation D en remplaçant le chlorure de 4-cyanobenzoyle par le chlorure de 4-phénylbenzoyle.

### PREPARATION F : 4-Bromo-N-(4-hydroxybutyl)benzamide

Le produit attendu est obtenu selon le procédé décrit dans la préparation D en remplaçant le chlorure de 4-cyanobenzoyle par le chlorure de 4-bromobenzoyle.

### PREPARATION G : 4-Fluoro-N-(4-hydroxybutyl)benzamide

Le produit attendu est obtenu selon le procédé décrit dans la préparation D en remplaçant le chlorure de 4-cyanobenzoyle par le chlorure de 4-fluorobenzoyle.

### PREPARATION H : N-(4-Hydroxybutyl)-2-trifluorométhyl-benzamide

Le produit attendu est obtenu selon le procédé décrit dans la préparation D en remplaçant le chlorure de 4-cyanobenzoyle par le chlorure de 2-trifluorométhylbenzoyle.

### PREPARATION I : N-(4-Bromophényl)-5-chloropentanamide

A 183 mmol (25 g) d'acide 5-chlorovalérique dans 250 ml de toluène portés à reflux, sont ajoutées goutte à goutte 366 mmol (26,7 ml) de chlorure de thionyle. Le milieu est agité à reflux jusqu'à la fin du dégagement gazeux, puis refroidi. Le solvant est évaporé. Le produit obtenu est alors remis en solution dans 250 ml de dichlorométhane, et la température est abaissée à 5°C. Une solution de 183 mmol (31,5 g) de 4-bromoaniline dans 100 ml de dichlorométhane et 183 mmol (25,5 ml) de triéthylamine sont ajoutées successivement. Le milieu réactionnel est agité 4 heures à 5°C puis 15 heures à température ambiante. Après hydrolyse, lavage de la phase organique par une solution d'acide chlorhydrique 1N, et évaporation des solvants, le produit attendu est obtenu et utilisé sans purification.

### PREPARATION J : 4-Phénylbenzaldéhyde

A 54 mmol d'alcool 4-phénylbenzylique dans 400 ml de dichlorométhane, sont ajoutées 65 mmole de dichromate de 4-benzyl-pyridinium. Le milieu réactionnel est agité 2 h à température ambiante. 600 ml d'un mélange éther éthylique/hexane dans un rapport un pour un sont ajoutés. Après une agitation de 30 min, le milieu est filtré. Le filtrat est lavé une fois par 100 ml d'une solution 1 N d'acide chlorhydrique. La phase organique est séchée sur sulfate de magnésium puis concentrée pour conduire au composé attendu.

### PREPARATION K : 4-Biphénylacétaldéhyde

Le produit attendu est obtenu selon de procédé décrit dans la préparation A en remplaçant le 2-(3-nitrophényl)éthanol par le 2-(4-biphényl)éthanol.

### PREPARATION L : 4-Bromophénylacétaldéhyde

Le produit attendu est obtenu selon le procédé décrit dans la préparation A en remplaçant le 2-(3-nitrophényl)éthanol par le 4-bromophényléthanol.

### PREPARATION M : 4-Nitrophénylacétaldéhyde.

Le produit attendu est obtenu selon le procédé décrit dans la préparation A en remplaçant le 2-(3-nitrophényl)éthanol par le 2-(4-nitrophényl)éthanol.

### PREPARATION N : 4-Bromo-N-(4-hydroxybutyl)benzène sulfonamide

Le produit attendu est obtenu selon le procédé décrit dans la préparation D en remplaçant le chlorure de 4-cyanobenzoyle par le chlorure de 4-bromobenzenesulfonyle.

### PREPARATION O : 4-Fluoro-N-(4-hydroxypropyl) benzamide

Le produit attendu est obtenu selon le procédé décrit dans la préparation G en remplaçant le 4-aminobutanol par le 3-aminopropanol.

### PREPARATION P : Chlorure de 4-acétylbenzoyle

A 600 mmol d'acide 4-acétylbenzoïque dans 150 ml de toluène, sont ajoutées 120 mmol de chlorure de thionyle. Le milieu réactionnel est porté au reflux pendant 3 h puis refroidi. Le solvant est évaporé. Le produit est utilisé sans autre traitement.

### PREPARATION Q : 4-Cyano-N-(4-hydroxybutyl)-1-méthoxy-2-naphtylamide

### Stade a : 2-(1-Hydroxy)naphtoate de méthyle

A 500 mmol d'acide 1-hydroxy-2-naphtoïque sont ajoutées 500 mmol d'*o*-méthyl caprolactime. Le milieu est chauffé à 85°C pendant 15 h, puis refroidi. Après avoir ajouté 50 ml d'éther éthylique, ramené le pH à une valeur de 9-10, le milieu est extrait à l'éther éthylique. Les phases organiques regroupées sont séchées sur sulfate de magnésium. Le produit attendu est précépité dans l'éther isopropylique et isolé après filtration.

### Stade b : 2-(4-Bromo-1-hydroxy)naphtoate de méthyle

440 mmol de brome sont additionnées lentement à 370 mmol du composé obtenu au stade a. Le milieu est agité 3 h à température ambiante avant d'être dilué avec de l'eau. Le précipité formé est lavé à l'eau, filtré et séché pour conduire au produit attendu.

### Stade c : 2-(4-Bromo-1-méthoxy)naphtoate de méthyle

350 mmol du composé obtenu au stade b sont ajoutées à 530 mmol de carbonate de potassium dans 2 I d'acétone. 590 mmol de diméthyl sulfate sont ensuite additionnées. Le milieu réactionnel est porté au reflux pendant 4 h puis filtré. Le filtrat est concentré. Le résidu est repris dans l'éther isopropylique et le précipité formé est filtré pour conduire au composé attendu.

### Stade d : 2-(4-Cyano-1-méthoxy)naphtoate de méthyle

110 mmol du composé obtenu au stade précédent, 70 mmol de cyanure de zinc et 7 mmol de tétrakis(triphénylphosphine)palladium sont ajoutés à 120 ml de diméthylformamide. Le milieu réactionnel est porté à 80°C pendant 5 h puis refroidi et dilué par du dichlorométhane. La phase organique est lavée par une solution 2 N d'ammoniaque puis séchée sur sulfate de magnésium. Le produit attendu est obtenu après purification par chromatographie sur gel de silice.

### Stade e : Acide 2-(4-cyano-1-méthoxy)naphtoïque

32 mmol du composé obtenu au stade précédent sont mises en solution dans 80 ml d'eau plus 80 ml de tétrahydrofurane. 36 mmol d'hydroxyde de lithium sont alors ajoutées. Après 2 h, le tétrahydrofurane est évaporé. La phase aqueuse est lavée à l'éther éthylique puis ramenée à pH = 3. Le précipité obtenu est filtré et lavé à l'eau jusqu'à pH neutre, puis séché, pour conduire au produit attendu.

### Stade f : Chlorure de l'acide 2-(4-cyano-1-méthoxy)naphtoïque

Le produit attendu est obtenu selon le procédé décrit dans la préparation P en remplaçant l'acide 4-acétyl benzoïque par le composé obtenu au stade précédent.

### Stade g: 4-Cyano-N-(4-hydroxybutyl)-1-méthoxy-2-naphtylamide

Le produit attendu est obtenu selon le procédé décrit dans la préparation D en remplaçant le chlorure de 4-cyanobenzoyle par le composé obtenu au stade précédent.

### PREPARATION R : 4-Fluoro-N-(2-hydroxyéthyl)benzamide

Le produit attendu est obtenu selon le procédé décrit dans la préparation G en remplaçant le 4-aminobutanol par le 2-aminoéthanol.

### PREPARATION S : 4-Phényl-N-(2-hydroxyéthyl)benzamide

Le produit attendu est obtenu selon le procédé décrit dans la préparation E en remplaçant le 4-aminobutanol par le 2-aminoéthanol.

### PREPARATION T : 4-Méthoxy-N(4-hydroxybutyl)benzène sulfonamide

Le produit attendu est obtenu selon le procédé décrit dans la préparation D en remplaçant le chlorure de 4-cyanobenzoyle par le chlorure de 4-méthoxybenzènesulfonyle.

### PREPARATION U : 4-Nitro-N-(4-hydroxybutyl)benzène sulfonamide

Le produit attendu est obtenu selon le procédé décrit dans la préparation D en remplaçant le chlorure de 4-cyanobenzoyle par le chlorure de 4-nitrobenzènesulfonyle.

### PREPARATION V : 4-Bromo-N-(3-hydroxypropyl)benzène sulfonamide

Le produit attendu est obtenu selon le procédé décrit dans la préparation N en remplaçant le 4-aminobutanol par le 3-aminopropanol.

### PREPARATION W : 4-Nitro-N-(3-hydroxypropyl)benzènesulfonamide

Le produit attendu est obtenu selon le procédé décrit dans la préparation U en remplaçant le 4-aminobutanol par le 3-aminopropanol.

### PREPARATION X : 4-Bromo-N-(2-hydroxyéthyl)benzènesulfonamide

Le produit attendu est obtenu selon le procédé décrit dans la préparation N en remplaçant le 4-aminobutanol par le 2-aminoéthanol.

### PREPARATION Y : 4-Méthoxy-N-(2-hydroxyéthyl)benzènesulfonamide

Le produit attendu est obtenu selon le procédé décrit dans la préparation T en remplaçant le 4-aminobutanol par le 2-aminoéthanol.

### EXEMPLE 1 : (4aα,10bβ)-9-Cyano-4-propyl-1,3,4,4a,5,10b-hexahydro-2H-chroméno[3,4-b]pyridine, chlorhydrate

### Stade a : Méthane sulfonate de (4aα, 10bβ)-9-hydroxy-4-propyl-1,3,4,4a,5,10b-hexahydro-2H-chroméno[3,4-b]pyridine

A 6,4 mmol de 9-hydroxy-4-propyl-1,3,4,4a,5,10b-hexahydro-2*H*-chroméno[3,4-*b*] pyridine (décrit dans J. Med. Chem., 1989, 32, 720-7) en solution dans 100 ml de dichlorométhane, refroidie à -30°C, sont ajoutées successivement 9,7 mmol de 2,6-lutidine, 1,3 mmol de 4-diméthylaminopyridine et 9,7 mmol d'anhydride triflique goutte à goutte. Après 30 minutes d'agitation, le milieu est hydrolysé à l'aide d'une solution saturée de NaCI. La phase organique est ensuite décantée, puis concentrée, et le produit attendu est purifié par chromatographie sur gel de silice.

### Stade b : (4aα,10bβ)-9-Cyano-4-propyl-1,3,4,4a,5,10b-hexahydro-2H-chroméno[3,4-b]pyridine, chlorhydrate

A 2,6 mmol du composé préparé au stade précédent, dans 20 ml de 1,2-dichloroéthane, sont ajoutées successivement 5,4 mmol de cyanure de tributylétaim, 7,8 mmol de chlorure de lithium et 2,6 mmol de tétrakis(triphénylphosphine)palladium. Le milieu réactionnel est porté à reflux pendant 18 heures. Après refroidissement et hydrolyse à l'aide d'une solution saturée de fluorure de potassium, le milieu est filtré. La phase organique est décantée et concentrée. Le produit attendu est obtenu après purification par chromatographie sur gel de silice.

Le chlorhydrate correspondant est obtenu par action d'une solution titrée d'acide chrlohydrique dans l'éthanol.

| Microanalyse élémentaire : | | | | |
|---|---|---|---|---|
| | C | H | Cl | N |
| % calculé | 65,63 | 7,23 | 12,11 | 9,57 |
| % trouvé | 65,28 | 7,06 | 11,77 | 9,30 |

### EXEMPLE 2 : (3aα,9bβ)-8-Cyano-2-phénéthyl-1,2,3,3a,4,9b-hexahydrochroméno[3,4-c]pyrrole, chlorhydrate

A 5 mmol de (3aα, 9bβ)-8-cyano-1,2,3,3a,4,9b-hexahydrochroméno[3,4-*c*] pyrrole (décrit dans la demande EP 691 342) dans 50 ml d'acétonitrile, sont ajoutées 10 mmol de carbonate de potassium. Le milieu réactionnel est agité à température ambiante pendant 15 minutes. 0,5 mmol d'iodure de potassium et 5 mmol de bromure de phénéthyle en solution dans 50 ml d'acétonitrile sont ajoutées successivement. Le milieu réactionnel est ensuite porté à reflux pendant 15 heures. Après hydrolyse et extraction par du dichlorométhane, les phases organiques sont rassemblées et concentrées. Le produit attendu est obtenu après purification par chromatographie sur gel de silice en utilisant comme éluant un mélange dichlorométhane / méthanol, 95/5. Le produit est transformé en chlorhydrate correspondant à l'aide d'une solution d'éthanol saturée en acide chlorhydrique.

| Microanalyse élémentaire : | | | | |
|---|---|---|---|---|
| | C | H | Cl | N |
| % calculé | 70,48 | 6,21 | 10,40 | 8,22 |
| % trouvé | 70,42 | 6,24 | 10,45 | 7,78 |

### EXEMPLE 3 : (3aα,9bβ)-8-Cyano-2-[2-(3-méthylsulfonylaminophényl)-éthyl]-1,2,3, 3a,4,9b-hexahydrochroméno[3,4-c]pyrrole, chlorhydrate

### Stade a : (3aα,9bβ)-8-Cyano-2-[2-(3-nitrophényl)-éthyl]-1,2,3,3a,4,9b-hexahydro chroméno[3,4-c]pyrrole

A 60 mmol du composé décrit dans la préparation A dans 500 ml de 1,2-dichloroéthane, sont ajoutées 30 mmol de (3aα, 9bβ)-8-cyano-1,2,3,3a,4,9b-hexahydrochroméno[3,4-c] pyrrole, (décrit dans la demande EP 691 342), puis 30 mmol d'acide acétique. Après 10 minutes, 42 mmol de triacétoxyborohydrure de sodium sont ajoutées. Le milieu réactionnel est agité à température ambiante pendant 15 heures, puis lavé par une solution saturée d'hydrogénocarbonate de sodium. La phase organique est décantée, séchée et concentrée. Le produit attendu est obtenu après purification par chromatographie sur gel de silice en utilisant comme éluant un mélange toluène/éthanol, 97/3.

### Stade b : (3aα,9bβ)-8-Cyano-2-[2-(3-aminophényl)-éthyl]-1,2,3,3a,4,9b-Hexahydrochroméno[3,4-c]pyrrole

A 9 mmol du composé obtenu au stade précédent dans 300 ml d'éthanol, sont ajoutées 0,6 g de Palladium sur charbon à 10 %. Le milieu réactionnel est mis sous atmosphère d'hydrogène pendant 1 h 30. Le produit attendu est obtenu après filtration du catalyseur et concentration du filtrat.

### Stade c : (3aα, 9bβ)-8-Cyano-2-[2-(3-méthylsulfonylaminophényl)-éthyl]-1,2,3, 3a,4,9b-hexahydrochroméno[3,4-c]pyrrole, chlorhydrate

A 10 mmol de chlorure de méthanesulfonyle dans 15 ml de dichlorométhane, à - 5°C, sont ajoutées 9 mmol du composé obtenu au stade b, en solution dans le dichlorométhane. Après 15 heures d'agitation à température ambiante, le milieu est traité par la soude 1N. La phase organique est décantée, séchée et concentrée. Le produit attendu est obtenu après purification par chromatographie sur gel de silice en utilisant comme éluant un mélange dichlorométhane/méthanol, 95/5. Le chlorhydrate correspondant est obtenu par action d'une solution titrée d'acide chlorhyrdrique dans l'éthanol.

| Microanalyse élémentaire : | | | | | |
|---|---|---|---|---|---|
| | C | H | Cl | N | S |
| % calculé | 58,12 | 5,57 | 8,17 | 9,68 | 7,39 |
| % trouvé | 58,56 | 5,85 | 8,25 | 9,37 | 6,98 |

### EXEMPLE 4 : (3aα,9bβ)-2-[2-(4-Acétylaminophényl)-éthyl]-8-cyano-1,2,3,3a, 4,9b-hexahydrochroméno[3,4-c]pyrrole, chlorhydrate

Le produit attendu est obtenu selon le procédé décrit dans l'Exemple 2, en remplaçant le bromure de phénéthyle par le composé décrit dans la Préparation B.

| Microanalyse élémentaire : | | | | |
|---|---|---|---|---|
| | C | H | Cl | N |
| % calculé | 66,41 | 6,08 | 8,91 | 10,56 |
| % trouvé | 66,35 | 6,10 | 8,89 | 10,25 |

### EXEMPLE 5 : (3aα,9bβ)-8-Cyano-2-[2-(2-oxo-2,3-dihydro-1H-indol-5-yl)-éthyl]-1,2,3,3a,4,9b-hexahydrochroméno[3,4-c]pyrrole, chlorhydrate

Le produit attendu est obtenu selon le procédé décrit dans l'Exemple 2, en remplaçant le bromure de phénéthyle par le composé décrit dans la Préparation C.

| Microanalyse élémentaire : | | | | |
|---|---|---|---|---|
| | C | H | Cl | N |
| % calculé | 66,75 | 5,60 | 8,96 | 10,61 |
| % trouvé | 66,24 | 5,77 | 8,51 | 10,45 |

### EXEMPLE 6 : (3aα,9bβ)-2-(4-Bromobenzyl)-8-cyano-1,2,3,3a,4,9b-hexahydrochroméno[3,4-c]pyrrole, chlorhydrate

Le produit attendu est obtenu selon le procédé décrit dans l'Exemple 2, en remplaçant le bromure de phénéthyle par le bromure de 4-bromobenzyle.

| Microanalyse élémentaire : | | | | |
|---|---|---|---|---|
| | C | H | Cl | N |
| % calculé | 56,25 | 4,47 | 8,74 | 6,90 |
| % trouvé | 56,50 | 4,74 | 8,37 | 6,45 |

### EXEMPLE 7 : (3aα,9bβ)-2-(4-Acétylaminobenzyl)-8-cyano-1,2,3,3a,4,9b-hexahydrochroméno[3,4-c]pyrrole, chlorhydrate

Le produit attendu est obtenu en utilisant le procédé décrit dans l'exemple 3, stade a, en remplaçant le produit de la préparation A par le 4-acétylaminobenzaldéhyde.

### EXEMPLE 8 : (3aα, 9bβ)-8-Cyano-2-(4-fluorobenzyl)-1,2,3,3a,4,9b-hexahydrochroméno[3,4-c]pyrrole, chlorhydrate

Le produit attendu est obtenu en utilisant le procédé décrit dans l'exemple 3, stade a, en remplaçant le produit de la préparation A par le 4-fluorobenzaldéhyde.

### EXEMPLE 9 : (3aα,9bβ)-8-Cyano-(3-nitrobenzyl)-1,2,3,3a,4,9b-hexahydrochroméno[3,4-c]pyrrole, chlorhydrate

Le produit attendu est obtenu en utilisant le procédé décrit dans l'exemple 2 en remplaçant le bromure de phénéthyle par le bromure de 3-nitrobenzyle.

### EXEMPLE 10 : (3aα,9bβ)-2-Benzyl-8-cyano-1,2,3,3a,4,9b-hexahydrochroméno-[3,4-c]pyrrole, chlorhydrate

Le produit est décrit dans l'exemple 29 de la demande EP 691 342.

### EXEMPLE 11 : (3aα,9bβ)-8-Aminocarbonyl-2-benzyl-1,2,3,3a,4,9b-hexahydrochroméno[3,4-c]pyrrole, chlorhydrate

A 15 mmol du composé décrit dans l'exemple 10 sont ajoutés 40 g d'acide polyphosphorique. Le milieu est chauffé à 140°C pendant 4 heures, puis versé sur un bain de glace. Le pH est amené à 12 par addition d'une solution aqueuse de NaOH concentrée. Après avoir ajouté 1 l d'eau et 300 ml de dichlorométhane, le milieu biphasique est agité 15 heures, puis décanté. Après extraction, la phase organique est séchée et concentrée. Le produit attendu est obtenu après purification par chromatographie sur gel de silice en utilisant comme éluant un mélange dichlorométhane/méthanol/ammoniaque, 96/4/0,4. Le chlorhydrate correspondant est obtenu par action d'une solution titrée d'acide chlorhydrique dans l'éthanol.

| Microanalyse élémentaire : | | | | |
|---|---|---|---|---|
| | C | H | Cl | N |
| % calculé | 66,18 | 6,14 | 10,28 | 8,12 |
| % trouvé | 65,64 | 6,16 | 10,01 | 7,84 |

### EXEMPLE 12 : (3aα,9bβ)-8-Cyano-2-propyl-1,2,3,3a,4,9b-hexahydrochroméno-[3,4-c] pyrrole, chlorhydrate

Le produit est décrit dans l'exemple 31 de la demande EP 691 342.

### EXEMPLE 13 : 4-Cyano-N-[4-((3aα,9bβ)-8-cyano-1,2,3,3a,4,9b-hexahydrochroméno[3,4-c]pyrrol-2-yl)-butyl]-benzamide, chlorhydrate

A 20 mmol de chlorure d'oxalyle en solution dans 48 ml de dichlorométhane à - 60°C, sont ajoutées 20 mmol de diméthylsulfoxyde en solution dans 12 ml de dichlorométhane. Une solution du composé décrit dans la préparation D, dans 32 ml de dichlorométhane est ajoutée lentement. Le milieu réactionnel est maintenu à - 60°C pendant 45 minutes, puis 50 mmol de triéthylamine sont ajoutées lentement. On laisse la réaction revenir à température ambiante, Après hydrolyse et extraction par le dichlorométhane, le phase organique est lavée par une solution saturée de chlorure de sodium et concentrée. Le produit obtenu est remis en solution dans 50 ml de 1,2-dichloroéthane puis sont ajoutées successivement 10 mmol de (3aα,9bβ)-8-cyano-1,2,3,3a,4,9b-hexahydrochroméno[3,4-c]pyrrole (décrit dans la demande EP 691 342) et 10 mmol d'acide acétique. Après 10 minutes d'agitation, 17,5 mmol de triacétoxyborohydrure de sodium sont ajoutées. L'agitation est maintenue pendant 15 heures, puis le milieu est hydrolysé par une solution saturée d'hydrogénocarbonate de sodium. Après extraction au dichlorométhane, le produit attendu est obtenu après purification par chromatographie sur gel de silice en utilisant comme éluant un mélange dichlorométhane/méthanol, 95/5. Le chlorhydrate correspondant est obtenu par action d'une solution d'éthanol saturée en acide chlorhydrique.

| Microanalyse élémentaire : | | | | |
|---|---|---|---|---|
| | C | H | Cl | N |
| % calculé | 65,97 | 5,77 | 8,11 | 12,82 |
| % trouvé | 66,67 | 5,79 | 8,16 | 12,73 |

### EXEMPLE 14 : N-[4-((3aα, 9bβ)-8-Cyano-1,2,3,3a,4,9b-hexahydrochroméno[3,4-c] pyrrol- 2-yl)-butyl]-4-phényl-benzamide, chlorhydrate

Le produit attendu est obtenu selon le procédé décrit dans l'exemple 7, en remplaçant le produit de la préparation D par le composé décrit dans la préparation E.

| Microanalyse élémentaire : | | | | |
|---|---|---|---|---|
| | C | H | Cl | N |
| % calculé | 71,37 | 6,20 | 7,26 | 8,61 |
| % trouvé | 71,46 | 6,17 | 7,54 | 8,51 |

### EXEMPLE 15 : 4-Bromo-N-[4-((3aα,9bβ)-8-cyano-1,2,3,3a,4,9b-hexahydrochroméno[3,4-c]pyrrol-2-yl)-butyl]-benzamide, chlorhydrate

Le produit attendu est obtenu selon le procédé décrit dans l'exemple 13 en remplaçant le produit de la préparation D par le composé décrit dans la préparation F.

| Microanalyse élémentaire : | | | | | |
|---|---|---|---|---|---|
| | C | H | Br | Cl | N |
| % calculé | 56,28 | 5,13 | 16,28 | 7,22 | 8,56 |
| % trouvé | 56,10 | 5,03 | 16,00 | 7,17 | 8,49 |

### EXEMPLE 16 : N-[4-((3aα, 9bβ)-8-Cyano-1,2,3,3a,4,9b-hexahydrochroméno[3,4-c] pyrrol-2-yl)-butyl]-4-fluoro-benzamide, chlorhydrate

Le produit attendu est obtenu selon le procédé décrit dans l'exemple 13 en remplaçant le produit de la préparation D par le composé décrit dans la préparation G.

| Microanalyse élémentaire : | | | | |
|---|---|---|---|---|
| | C | H | Cl | N |
| % calculé | 64,26 | 5,86 | 8,25 | 9,77 |
| % trouvé | 64,08 | 5,93 | 8,69 | 9,62 |

### EXEMPLE 17 : N-[4-((3aα,9bβ)-8-Cyano-1,2,3,3a,4,9b-hexahydrochroméno[3,4-c] pyrrol-2-yl)-butyl]-2-trifluorométhyl-benzamide, chlorhydrate

Le produit attendu est obtenu selon le procédé décrit dans l'exemple 13 en remplaçant le produit de la préparation D par le composé décrit dans la préparation H.

| Microanalyse élémentaire : | | | | |
|---|---|---|---|---|
| | C | H | Cl | N |
| % calculé | 60,06 | 5,25 | 7,39 | 8,76 |
| % trouvé | 60,47 | 5,15 | 7,36 | 8,87 |

### EXEMPLE 18 : N-(4-Bromophényl)-5-[(3aα,9bβ)-8-cyano-1,2,3,3a,4,9b-hexahydrochroméno[3,4-c]pyrrol-2-yl]pentanamide, chlorhydrate

Le produit attendu est obtenu selon le procédé décrit dans l'Exemple 2, en remplaçant le bromure de phénéthyle par le composé décrit dans la Préparation 1.

### EXEMPLE 19 : (3aα,9bβ)-8-Aminocarbonyl-2-[2-(2-oxo-2,3-dihydro-1H-indol-5-yl) éthyl]-1,2,3,3a,4,9b-hexahydrochroméno[3,4-c]pyrrole, chlorhydrate

Le produit attendu est obtenu selon le procédé décrit dans l'exemple 11 en remplaçant le composé de l'exemple 10 par le produit décrit dans l'exemple 5.

| Microanalyse élémentaire : | | | | |
|---|---|---|---|---|
| | C | H | Cl | N |
| % calculé | 63,52 | 5,92 | 8,60 | 9,98 |
| % trouvé | 63,84 | 5,84 | 8,52 | 10,15 |

### EXEMPLE 20 : (3aα,9bβ)-8-Cyano-2-(4-phénylbenzyl)-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par celui de la préparation J. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 74,62 | 5,86 | 8,61 | 6,85 |
| % trouvé | 74,52 | 5,50 | 8,78 | 6,95 |

### EXEMPLE 21 : (3aα, 9bβ)-8-Cyano-2-[4-(4-fluorophényl)benzyl]-1,2,3,3a,4,9b-hexahydrochroméno[3,4-c] pyrrole, chlorhydrate

A 100 mg de tétrakis-triphénylphosphine palladium dans 20 ml de toluène sont ajoutés, 2,7 mmole du composé de l'exemple 6 sous sa forme base, 2,7 ml d'une solution 2 M de carbonate de sodium et 3 mmol d'acide 4-fluorophényl boronique en solution dans 2 ml d'éthanol. Le milieu réactionnel est porté au reflux pendant 3 h puis refroidi. 0,2 ml d'une solution d'eau oxygénée à 30 % dans l'eau est ajouté. Après 30 min d'agitation, 50 ml d'éther éthylique sont ajoutés. Le milieu est décanté, la phase aqueuse est extraite une fois à l'éther. Les phases organiques sont rassemblées et lavées par une solution saturée de chlorure de sodium puis séchées sur sulfate de magnésium. Le produit attendu est obtenu après purification par chromatographie sur gel de silice. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 71,19 | 5,36 | 8,48 | 6,69 |
| % trouvé | 71,34 | 5,27 | 8,42 | 6,66 |

### EXEMPLE 22 : (3aα,9bβ)-8-Cyano-2-[2-(4-biphényl)éthyl]-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par celui de la préparation K. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 74,67 | 6,06 | 8,51 | 6,76 |
| % trouvé | 74,90 | 6,04 | 8,50 | 6,72 |

### EXEMPLE 23 : (3aα,9bβ)-8-Cyano-2-[2-(4-bromophényl)éthyl]-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par celui de la préparation L, sans ajouter l'acide acétique. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 56,90 | 4,82 | 8,19 | 6,40 |
| % trouvé | 57,23 | 4,80 | 8,45 | 6,67 |

### EXEMPLE 24 : (3aα,9bβ)-8-Cyano-2-[2-(4'-fluoro-4-biphényl)éthyl]-1,2,3,3a,4,9b-hexahydrochroméno[3,4-c]pyrrole, chlorhydrate.

Le produit attendu est obtenu selon le procédé décrit dans l'exemple 21 en remplaçant le composé de l'exemple 6 par celui de l'exemple 23 sous sa forme base.

| Microanalyse élémentaire : | | | | |
|---|---|---|---|---|
| | C | H | Cl | N |
| % calculé | 71,62 | 5,66 | 7,98 | 6,42 |
| % trouvé | 71,79 | 5,56 | 8,15 | 6,44 |

### EXEMPLE 25 : (3aα,9bβ)-8-Cyano-2-(4-phényloxybenzyl)-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par le 4-phényloxybenzaldéhyde. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 71,46 | 5,58 | 8,58 | 6,77 |
| % trouvé | 71,68 | 5,53 | 8,46 | 6,69 |

### EXEMPLE 26 : (3aα,9bβ)-2-(4-Benzyloxybenzyl)-8-cyano-1,2,3,3a,4,9b-hexahydro chroméno[3,4-c]pyrrole, chlorhydrate

Le produit attendu est obtenu selon le procédé décrit dans l'exemple 3, stade a en remplaçant le produit de la préparation A par le 4-benzyloxybenzaldéhyde, sans ajouter l'acide acétique. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 71,90 | 5,91 | 8,22 | 6,42 |
| % trouvé | 72,13 | 5,82 | 8,19 | 6,47 |

### EXEMPLE 27 : (3aα,9bβ)-8-Cyano-2-(2-furylméthyl)-1,2,3,3a,4,9b-hexahydrochroméno[3,4-c]pyrrole, chlorhydrate.

Le produit attendu est obtenu selon le procédé décrit dans l'exemple 3, stade a en remplaçant le produit de la préparation A par le 2-furaldéhyde, sans ajouter l'acide acétique. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 64,32 | 5,48 | 11,33 | 8,53 |
| % trouvé | 64,46 | 5,41 | 11,19 | 8,84 |

### EXEMPLE 28 : (3aα,9bβ)-2-(3-Bromobenzyl)-8-cyano-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par le 3-bromobenzaldéhyde, sans ajouter l'acide acétique. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 56,06 | 4,43 | 8,62 | 6,62 |
| % trouvé | 56,25 | 4,47 | 8,74 | 6,90 |

### EXEMPLE 29 : (3aα,9bβ)-2-(2-Bromobenzyl)-8-cyano-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par le 2-bromobenzaldéhyde, sans ajouter l'acide acétique. Le produit est transformé en chlorhydrate correspondant à l'aide d'une solution titrée d'acide chlorhydrique dans l'éthanol.

| Microanalyse élémentaire : | | | | |
|---|---|---|---|---|
| | C | H | Cl | N |
| % calculé | 55,95 | 4,48 | 8,46 | 6,61 |
| % trouvé | 56,25 | 4,47 | 8,74 | 6,90 |

### EXEMPLE 30 : (3aα,9bβ)-8-Cyano-2-[4-(2-thiényl)benzyl]-1,2,3,3a,4,9b-hexahydro chroméno[3,4-c]pyrrole, chlorhydrate

Le produit attendu est obtenu selon le procédé décrit dans l'exemple 21, en remplaçant l'acide 4-fluorophényl boronique par l'acide 2-thiénylboronique.

| Microanalyse élémentaire : | | | | |
|---|---|---|---|---|
| | C | H | Cl | N |
| % calculé | 67,83 | 5,21 | 8,25 | 6,83 |
| % trouvé | 67,55 | 5,18 | 8,67 | 6,85 |

### EXEMPLE 31 : (3aα, 9bβ)-8-Cyano-2-{4-[2-(4-méthoxyphényl)vinyl]benzyl}-1,2,3, 3a,4,9b-hexahydrochroméno[3,4-c]pyrrole, chlorhydrate

4 mmol de 4-méthoxystyrène puis 3,9 ml de triéthylamine sont ajoutés à 4 mmol du composé de l'exemple 6 sous sa forme base dans 40 ml de DMF. 0,2 mmol de diacétate de palladium et 0,8 mmol de tri-ortho-tolylphosphine sont ajoutées au milieu réactionnel. Le milieu est porté à 100°C pendant 3 h. Après refroidissement et addition d'eau, le milieu est décanté. La phase aqueuse est extraite par deux fois 50 ml d'éther. Les phases organiques sont rassemblées et séchées par du sulfate de magnésium. Le produit attendu est obtenu après purification par chromatographie sur gel de silice. Le chlorhydrate correspondant est obtenu par action d'une solution titrée d'acide chlorhydrique dans l'éthanol.

| Microanalyse élémentaire : | | | | |
|---|---|---|---|---|
| | C | H | Cl | N |
| % calculé | 72,90 | 6,00 | 7,41 | 6,14 |
| % trouvé | 73,27 | 5,93 | 7,72 | 6,10 |

### EXEMPLE 32 : (3aα, 9bβ)-8-Cyano-2-{4-[2-(4-méthoxyphényl)ethyl]benzyl}-1,2,3, 3a,4,9b-hexahydrochroméno[3,4-c]pyrrole, chlorhydrate

4 mmol du composé de l'exemple 31 sont mises en solution dans 150 ml d'éthanol. Après addition de 100 mg de palladium sur charbon, le milieu est agité sous pression atmosphérique d'hydrogène. Après 2 h à température ambiante, le milieu réactionnel est filtré, le filtrat est ensuite évaporé, pour conduire au composé attendu.

| Microanalyse élémentaire : | | | | |
|---|---|---|---|---|
| | C | H | Cl | N |
| % calculé | 72,82 | 6,39 | 7,81 | 6,01 |
| % trouvé | 72,95 | 6,34 | 7,69 | 6,07 |

### EXEMPLE 33 : (3aα,9bβ)-8-Cyano-2-{2-[4-(2-thiényl)phényl]-éthyl}-1,2,3,3a,4,9b-hexahydrochroméno[3,4-c]pyrrole, chlorhydrate

Le produit attendu est obtenu selon le procédé décrit dans l'exemple 30 en remplaçant le composé de l'exemple 6 par celui de l'exemple 23 sous sa forme base.

| Microanalyse élémentaire : | | | | |
|---|---|---|---|---|
| | C | H | Cl | N |
| % calculé | 67,36 | 5,59 | 8,50 | 6,68 |
| % trouvé | 67,22 | 5,64 | 8,62 | 6,81 |

### EXEMPLE 34 : (3aα,9bβ)-8-Cyano-2-[2-(4-méthylsulfonylaminophényl)-éthyl]-1,2,3,3a,4,9b-hexahydrochroméno[3,4-c]pyrrole, chlorhydrate

Le produit attendu est obtenu selon le procédé décrit dans l'exemple 3 en remplaçant au stade a le composé de la préparation A par le dérivé décrit dans la préparation M.

| Microanalyse élémentaire : | | | | |
|---|---|---|---|---|
| | C | H | Cl | N |
| % calculé | 58,12 | 5,57 | 8,17 | 9,68 |
| % trouvé | 52,79 | 5,75 | 8,44 | 9,32 |

### EXEMPLE 35 : 4-Bromo-N-{4-[(3aα,9bβ)-8-cyano-1,2,3,3a,4,9b-hexahydrochroméno[3,4-c]pyrrol-2-yl]butyl}benzène sulfonamide, chlorhydrate

Le produit attendu est obtenu selon le procédé décrit dans l'exemple 13 en remplaçant le produit de la préparation D par le composé décrit dans la préparation N.

### EXEMPLE 36 : N-[4-((3aα,9bβ)-8-Cyano-1,2,3,3a,4,9b-hexahydrochroméno[3,4-c] pyrrol-2-yl)propyl]-4-fluorobenzamide, chlorhydrate

Le produit attendu est obtenu selon le procédé décrit dans l'exemple 13 en remplaçant le produit de la préparation D par le composé décrit dans la préparation O.

| Microanalyse élémentaire : | | | | |
|---|---|---|---|---|
| | C | H | Cl | N |
| % calculé | 62,92 | 5,67 | 8,45 | 9,80 |
| % trouvé | 63,54 | 5,57 | 8,52 | 10,10 |

### EXEMPLE 37 : 4-Acétyl-N-{4-[2-(8-cyano-1,2,3,3a,4,9b-hexahydrochroméno [3,4-c]pyrrol-2-yl)éthyl)phényl]benzamide, chlorhydrate

### Stade a : (3aα,9bβ)-8-Cyano-2-[2-(4-nitrophényl)-éthyl]-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par celui de la préparation M.

### Stade b : (3aα,9bβ)-8-Cyano-2-[2-(4-aminophényl)-éthyl]-1,2,3,3a,4,9b-hexahydrochroméno[3,4-c]pyrrole

Le produit attendu est obtenu selon le procédé décrit dans l'exemple 3, stade b.

### Stade c : 4-Acétyl-N-{4-[2-(8-cyano-1,2,3,3a,4,9b-hexahydrochroméno[3,4-c] pyrrol-2-yl)éthyl]phényl}benzamide, chlorhydrate

Le produit attendu est obtenu selon le procédé décrit dans l'exemple 3, stade c en remplaçant le chlorure de méthane sulfonyle par le composé de la préparation P.

### EXEMPLE 38 : 4-Cyano-1-méthoxy-N-[4-(8-cyano-1,2,3,3a,4,9b-hexahydro chroméno[3,4-c]pyrrol-2-yl)butyl]-2-naphtylamide, chlorhydrate

Le produit attendu est obtenu selon le procédé décrit dans l'exemple 13 en remplaçant le produit de la préparation D par le composé décrit dans la préparation Q.

| Microanalyse élémentaire : | | | | |
|---|---|---|---|---|
| | C | H | Cl | N |
| % calculé | 67,42 | 5,61 | 6,78 | 10,52 |
| % trouvé | 67,37 | 5,65 | 6,86 | 10,84 |

### EXEMPLE 39 : 4-Fluoro-N-[2-((3aα,9bβ)-8-cyano-1,2,3,3a,4,9b-hexahydrochroméno[3,4-c]pyrrol-2-yl)éthyl]benzamide, chlorhydrate

Le produit attendu est obtenu selon le procédé décrit dans l'exemple 13 en remplaçant le produit de la préparation D par le composé décrit dans la préparation R.

### EXEMPLE 40: 4-Phényl-N-[2-((3aα,9bβ)-8-cyano-1,2,3,3a,4,9b-hexahydrochroméno[3,4-c]pyrrol-2-yl)éthyl]benzamide, chlorhydrate

Le produit attendu est obtenu selon le procédé décrit dans l'exemple 13 en remplaçant le produit de la préparation D par le composé décrit dans la préparation S.

### EXEMPLE 41 : N-{4-[(3aα,9bβ)-8-cyano-1,2,3,3a,4,9b-hexahydrochroméno[3,4-c] pyrrol-2-yl]butyl}-4-méthoxybenzène sulfonamide, chlorhydrate

Le produit attendu est obtenu selon le procédé décrit dans l'exemple 13 en remplaçant le produit de la préparation D par le composé décrit dans la préparation T.

### EXEMPLE 42 : N-{4-[(3aα,9bβ)-8-Cyano-1,2,3,3a,4,9b-hexahydrochroméno[3,4-c] pyrrol-2-yl]butyl}-4-nitrobenzènesulfonamide, chlorhydrate

Le produit attendu est obtenu selon le procédé décrit dans l'exemple 13 en remplaçant le produit de la préparation D par le composé décrit dans la préparation U.

### EXEMPLE 43 : 4-Bromo-N-{3-[(3aα,4bβ)-8-cyano-1,2,3,3a,4,9b-hexahydro chroméno[3,4-c]pyrrol-2-yl]propyl}benzènesulfonamide, chlorhydrate

Le produit attendu est obtenu selon le procédé décrit dans l'exemple 13 en remplaçant le produit de la préparation D par le composé décrit dans la préparation V.

### EXEMPLE 44 : N-{3-[(3aα,9bβ)-8-Cyano-1,2,3,3a,4,9b-hexahydrochroméno[3,4-c] pyrrol-2-yl]propyl}-4-nitrobenzènesulfonamide, chlorhydrate

Le produit attendu est obtenu selon le procédé décrit dans l'exemple 13 en remplaçant le produit de la préparation D par le composé décrit dans la préparation W.

### EXEMPLE 45 : 4-Bromo-N-{2-[(3aα,9bβ)-8-cyano-1,2,3,3a,4,9b-hexahydrochroméno[3,4-c]pyrrol-2-yl]éthyl}benzènesulfonamide, chlorhydrate

Le produit attendu est obtenu selon le procédé décrit dans l'exemple 13 en remplaçant le produit de la préparation D par le composé décrit dans la préparation X.

### EXEMPLE 46 : N-{2-[(3aα,9bβ)-8-Cyano-1,2,3,3a,4,9b-hexahydrochroméno[3,4-c] pyrrol-2-yl]éthyl}-4-méthoxybenzène sulfonamide, chlorhydrate

Le produit attendu est obtenu selon le procédé décrit dans l'exemple 13 en remplaçant le produit de la préparation D par le composé décrit dans la préparation Y.

### EXEMPLE 47 : (3aα,9bβ)-8-Cyano-2-(2-pyridylméthyl)-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par le 2-pyridylcarboxaldéhyde.

### EXEMPLE 48 : (3aα,9bβ)-2-[(6-[1,4]Benzodioxinyl)méthyl]-8-cyano-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par le 6-formyl[1,4]benzodioxine.

### EXEMPLE 49 : (3aα,9bβ)-2-[(5-Benzothiényl)méthyl]-8-cyano-1,2,3,3a,4,9b-hexahydrochroméno[3,4-c]pyrrole, chlorhydrate

Le produit attendu est obtenu selon le procédé décrit dans l'exemple 3, stade a en remplaçant le produit de la préparation A par le 5-formylbenzothiophène.

### EXEMPLE 50 : (3aα,9bβ)-8-Cyano-2-[2-(4-phénylsulfonylaminophényl)éthyl] 1,2,3,3a,4,9b-hexahydrochroméno[3,4-c]pyrrole, chlorhydrate

Le composé attendu est obtenu selon le procédé décrit dans l'exemple 3 en remplaçant au stade c le chlorure de méthanesulfonyle par le chlorure de benzène sulfonyle.

### EXEMPLE 51 : (4aα,10bβ)-4-(4-Bromobenzyl)-9-cyano-1,3,4,4a,5,10b-hexahydro-2H-chroméno[3,4-b]pyridine, chlorhydrate

Le produit attendu est obtenu selon le procédé décrit dans l'exemple 6 en utilisant comme produit de départ le 9-cyano-1,3,4,4a,5,10b-hexahydro-*2H*-chroméno[3,4-c] pyridine (ce dernier ayant été préparé à partir de l'analogue hydroxylé en position 9 décrit dans J. Med. Chem., 1989, 32, 720).

### EXEMPLE 52 : (4aα, 10bβ)-9-Cyano-4-[4-(4-fluorophényl)benzyl]-1,3,4,4a,5,10b-hexahydro-2H-chroméno[3,4-b]pyridine, chlorhydrate

Le produit attendu est obtenu selon le procédé décrit dans l'exemple 21 en utilisant comme produit de départ le composé décrit dans l'exemple 51.

### EXEMPLE 53 : (4aα,10bβ)-4-(4-Acétylaminobenzyl)-9-cyano-1,3,4,4a,5,10b-hexahydro-2H-chroméno[3,4-b]pyridine, chlorhydrate

Le produit attendu est obtenu selon le procédé décrit dans l'exemple 7 en utilisant comme produit de départ le 9-cyano-1,3,4,4a,5,10b-hexahydro-*2H*-chroméno[3,4-*c*] pyridine (ce dernier ayant été préparé à partir de l'analogue hydroxylé en position 9 décrit dans J. Med. Chem., 1989, 32, 720).

### EXEMPLE 54 : (4aα,10bβ)-4-[2-(4-Acétylaminophényl)éthyl]-9-cyano-1,3,4,4a,5, 10b-hexahydro-2H-chroméno[3,4-b]pyridine, chlorhydrate

Le produit attendu est obtenu selon le procédé décrit dans l'exemple 4 en utilisant comme produit de départ le 9-cyano-1,3,4,4a,5,10b-hexahydro-*2H*-chroméno[3,4-c] pyridine (ce dernier ayant été préparé à partir de l'analogue hydroxylé en position 9 décrit dans J. Med. Chem., 1989, 32, 720).

### EXEMPLE 55 : (4aα,10bβ)-9-Cyano-4-furylméhtyl-1,3,4,4a,5,10b-hexahydro-2H-chroméno[3,4-b]pyridine, chlorhydrate

Le produit attendu est obtenu selon le procédé décrit dans l'exemple 27 en utilisant comme produit de départ le 9-cyano-1,3,4,4a,5,10b-hexahydro-*2H*-chroméno[3,4-c] pyridine (ce dernier ayant été préparé à partir de l'analogue hydroxylé en position 9 décrit dans J. Med. Chem., 1989, 32, 720).

### EXEMPLE 56 : 4-Bromo-N-[4-((4aα,10bβ)-9-cyano-1,3,4,4a,5,10b-hexahydro-2H-chroméno[3,4-b]pyrid-4-yl)butyl]benzamide, chlorhydrate

Le produit attendu est obtenu selon le procédé décrit dans l'exemple 15 en utilisant comme produit de départ le 9-cyano-1,3,4,4a,5,10b-hexahydro-*2H*-chroméno[3,4-c] pyridine (ce dernier ayant été préparé à partir de l'analogue hydroxylé en position 9 décrit dans J. Med. Chem., 1989, 32, 720).

### EXEMPLE 57 : 4-Cyano-N-[4-((4aα,10bβ)-9-cyano-1,3,4,4a,5,10b-hexahydro-2H-chroméno[3,4-b]pyrid-4-yl)butyl]benzamide, chlorhydrate

Le produit attendu est obtenu selon le procédé décrit dans l'exemple 13 en utilisant comme produit de départ le 9-cyano-1,3,4,4a,5,10b-hexahydro-*2H*-chroméno[3,4-c] pyridine (ce dernier ayant été préparé à partir de l'analogue hydroxylé en position 9 décrit dans J. Med. Chem., 1989, 32, 720).

### EXEMPLE 58 : N-[4-((4aα,10bβ)-9-Cyano-1,3,4,4a,5,10b-hexahydro-2H-chroméno [3,4-b]pyrid-4-yl)butyl]-4-phénylbenzamide, chlorhydrate

Le produit attendu est obtenu selon le procédé décrit dans l'exemple 14 en utilisant comme produit de départ le 9-cyano-1,3,4,4a,5,10b-hexahydro-2H-chroméno[3,4-c] pyridine (ce dernier ayant été préparé à partir de l'analogue hydroxylé en position 9 décrit dans J. Med. Chem., 1989, 32, 720).

### EXEMPLE 59 : 4-Bromo-N-[4-((4aα,10bβ)-9-cyano-1,3,4,4a,5,10b-hexahydro-2H-chroméno[3,4-b]pyrid-4-yl)butyl]benzènesulfonamide, chlorhydrate

Le produit attendu est obtenu selon le procédé décrit dans l'exemple 35 en utilisant comme produit de départ le 9-cyano-1,3,4,4a,5,10b-hexahydro-*2H*-chroméno[3,4-c] pyridine (ce dernier ayant été préparé à partir de l'analogue hydroxylé en position 9 décrit dans J. Med. Chem., 1989, 32, 720).

### EXEMPLE 60 : Mesure de l'affinité in vitro aux récepteurs D₂ et D₃

L'affinité des composés de l'invention vis à vis des récepteurs D₂ et D₃ humains (exprimés indépendamment et de façon stable dans les cellules CHO) a été déterminée sur des préparations membranaires en utilisant le [¹²⁵I]-iodosulpiride comme radioligand (Sokoloff et al., référence citée). Les résultats sont exprimés en pKi.

Ces résultats mettent en évidence la sélectivité *in vitro* des composés de l'invention, pour les récepteurs D₃ vis à vis des récepteurs D2. C'est le cas notamment du composé de l'Exemple 4, pour lequel cette sélectivité est supérieure à 1,5 log.

### EXEMPLE 61 : Mise en évidence in vivo de la sélectivité pour les récepteurs D₃ vis à vis des récepteurs D₂

La sélectivité in *vivo* des composés de l'invention pour les récepteurs D₃ vis à vis des récepteurs D₂ a été démontrée, chez le rat, par la capacité de ces composés à moduler l'hypothermie induite par l'agoniste dopaminergique D₃, 7-OH-DPAT, le contrôle de la température corporelle étant sous la dépendance du récepteur D₃ post-synaptique (M. Millan, référence citée).

### Méthode

Les tests ont été réalisés sur des rats Witsar, mâles, de 200-250 g, placés en cage individuelle, avec accès libre à la nourriture et à l'eau. Les produits ont été solubilisés dans de l'eau distillée, à laquelle plusieurs gouttes d'acide lactique sont ajoutées. Les injections ont été effectuées par voie sous cutanée.

Dans un premier temps, le produit à tester ou le véhicule est injecté, puis les rats sont replacés dans leurs cages pendant 30 minutes. Dans un deuxième temps, les rats subissent une injection de 7-OH-DPAT ou de véhicule et sont replacés dans leurs cages. Trente minutes plus tard, la température rectale est mesurée (en utilisant un thermistroprobe digital, Millan et al., J. Pharmacol. Exp. Ther., 1993, 264, 1364-76), et la différence est déterminée par rapport aux valeurs basales (ΔT°C)

### Résultats

Il apparaît que les composés de l'invention sont capables de moduler de façon significative l'hypothermie induite par le 7-OH-DPAT (pour les dérivés les plus actifs, l'effet du ligand D₃ de référence est pratiquement annulé).

### EXEMPLE 62 : Composition pharmaceutique

| Formule de préparation pour 1000 comprimés dosés à 10 mg | |
|---|---|
| Composé de l'Exemple 4 | 10 g |
| Hydroxypropylcellulose | 2 g |
| Amidon de blé | 10 g |
| Lactose | 100 9 |
| Stéarate de magnésium | 3 g |
| Talc | 3 g |

## Revendications

1. Composés de formule (I) : 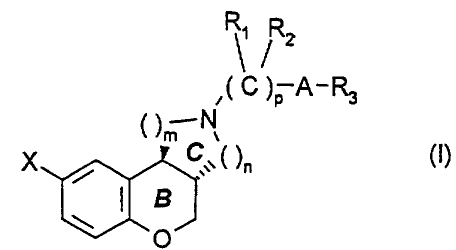 dans laquelle :
• ( ) représente un groupement -CH₂-,
• m est un entier tel que 0 ≤ m ≤ 3,
• n est un entier tel que 0 ≤ n ≤ 3 et 2 ≤ m+n ≤ 3,
• p est un entier tel que 1 ≤ p ≤ 6,
• la jonction entre les cycles *B* et *C* est de configuration trans,
• X représente un groupement cyano, ou un groupement -CO-NR₄R₅, R₄ et R₅ étant choisis parmi hydrogène, alkyle (C₁-C₆) linéaire ou ramifié, cycloalkyle (C₃-C₇), aryle éventuellement substitué,
• A représente une liaison σ ou un groupement choisi parmi -NR-CO-, -CO-NR-, -NR-SO₂, -SO₂-NR, dans lesquels R représente un atome d'hydrogène ou un groupement alkyle (C₁-C₆) linéaire ou ramifié,
• R₁ et R₂ représentent indépendamment un atome d'hydrogène ou un groupement alkyle (C₁-C₆) linéaire ou ramifié,
• R₃ représente :
- un atome d'hydrogène, un groupement phényle, naphtyle ou hétéroaryle, chacun de ces groupements étant éventuellement substitué par un ou plusieurs atomes d'halogènes, groupements alkyle (C₁-C₆) linéaire ou ramifié, alkoxy (C₁-C₆) linéaire ou ramifié, hydroxy, cyano, amino, nitro, carboxy, perhalogénoalkyle (C₁-C₆) linéaire ou ramifié, sulfonyle, acylamino, alkyl (C₁-C₆) sulfonyle linéaire ou ramifié, alkyle (C₁-C₆) sulfonylamino linéaire ou ramifié,
- un groupement aryle, ou hétéroaryle substitué par un groupement A'-Cy dans lequel A' représente une liaison σ ou un groupement alkylène (C₁-C₆) linéaire ou ramifié (dans lequel un atome de carbone peut être éventuellement remplacé par un atome d'oxygène ou de soufre), alkénylène (C₁-C₆) linéaire ou ramifié (dans lequel un atome de carbone peut être éventuellement remplacé par un atome d'oxygène ou de soufre), un groupement -NR-CO-, -CO-NR, -NR-SO₂-, SO₂-NR, dans lesquels R représente un atome d'hydrogène ou un groupement alkyle (C₁-C₆) linéaire ou ramifié, et Cy représente un groupement aryle éventuellement substitué, ou hétéroaryle éventuellement substitué,
- un groupement 2-indolinone-5-yl,
- ou bien un groupement aryloxy ou arylthio, à la condition que dans ce cas A représente une liaison σ,
étant entendu que :
• lorsque n est égal à 0, alors m est différent de 2,
• lorsque n est égal à 1, R₁ et R₂ représentent un hydrogène, A représente une liaison σ et p est égal à 1, alors R₃ est différent de phényle et pyridyle,
• lorsque n est égal à 1, R₁ et R₂ représentent un hydrogène, A représente une liaison σ, alors R₃ est différent d'un atome d'hydrogène,
• lorsque n est égal à 1, R₁ et R₂ représentent un hydrogène, A représente un groupement -NH-CO-, alors R₃ est différent d'un atome d'hydrogène, d'un groupement phényle, naphtyle, hétérocyclique choisi parmi thiényle, furyle, pyrrolyle, pyridyle, chacun de ces groupements étant éventuellement substitué par un ou plusieurs atomes d'halogène, ou groupements trihalogénométhyle, alkoxy ou hydroxy,
leurs énantiomères, diastéréoisomères, ainsi que leurs sels d'addition à un acide ou à une base pharmaceutiquement acceptable.

2. Composés de formule (I) selon la revendication 1 tel que m et n sont tous les deux égaux à 1, leurs énantiomères, diastéréoisomères, ainsi que leurs sels d'addition à un acide ou à une base pharmaceutiquement acceptable.

3. Composés de formule (I) selon la revendication 1 tel que m est égal à 3 et n est égal à 0, leurs énantiomères, diastéréoisomères, ainsi que leurs sels d'addition à un acide ou à une base pharmaceutiquement acceptable.

4. Composés de formule (I) selon la revendication 1 tel que X représente un groupement cyano, leurs énantiomères, diastéréoisomères, ainsi que leurs sels d'addition à un acide ou à une base pharmaceutiquement acceptable.

5. Composés de formule (I) selon la revendication 1 tel que A représente une liaison σ, leurs énantiomères, diastéréoisomères, ainsi que leurs sels d'addition à un acide ou à une base pharmaceutiquement acceptable.

6. Composés de formule (I) selon la revendication 1 tel que A représente un groupement -NR-CO-, ou NR-SO₂, leurs énantiomères, diastéréoisomères, ainsi que leurs sels d'addition à un acide ou à une base pharmaceutiquement acceptable.

7. Composés de formule (I) selon la revendication 1 tel que R₃ représente un groupement phényle éventuellement substitué, ou biphényle éventuellement substitué, leurs énantiomères, diastéréoisomères, ainsi que leurs sels d'addition à un acide ou à une base pharmaceutiquement acceptable.

8. Composés de formule (I) selon la revendication (I) tel que R₃ représente un groupement aryle substitué par un groupement A'-Cy dans lequel A' représente un groupement -NR-CO ou -NR-SO₂-, leurs énantiomères, diastéréoisomères, ainsi que leurs sels d'addition à un acide ou à une base pharmaceutiquement acceptable.

9. Composés de formule (I) selon la revendication 1 tel que X représente un groupement cyano, m et n sont tous les deux égaux à 1, R₁ et R₂ représentent chacun un atome d'hydrogène, et p vaut 4 tandis que A représente un groupement NHCO et R₃ un groupement phényle éventuellement substitué ou biphényle éventuellement substitué, ou bien p vaut 1 ou 2 tandis que A représente une liaison σ et R₃ un groupement phényle éventuellement substitué ou biphényle éventuellement substitué, leurs énantiomères, diastéréoisomères, ainsi que leurs sels d'addition à un acide ou à une base pharmaceutiquement acceptable.

10. Composé de formule (I) selon la revendication 1 qui est le (3aα,9bβ)-2-[2-(4-acétylaminophényl)-éthyl]-8-cyano-1,2,3,3a,4,9b-hexahydrochroméno[3,4-*c*]pyrrole, ses énantiomères ainsi que ses sels d'addition à un acide pharmaceutiquement acceptable.

11. Composé de formule (I) selon la revendication 1 qui est le N-[4-((3aα,9bβ)-8-cyano-1,2,3,3a,4,9b-hexahydrochroméno[3,4-*c*]pyrrol-2yl)butyl]-4-phénylbenzamide, ses énantiomères et ses sels d'addition à un acide pharmaceutiquement acceptable.

12. Composé de formule (I) selon la revendication 1 qui est le (3aα,9bβ)-8-cyano-2-[2-(2-oxo-2,3-dihydro-*1H*-indol-5-yl)éthyl]-1,2,3,3a,4,9b-hexahydrochroméno[3,4-*c*]pyrrole, ses énantiomères et ses sels d'addition à un acide pharmaceutiquement acceptable.

13. Composé de formule (I) selon la revendication 1 qui est le (3aα,9bβ)-8-cyano-2-(4-fluorobenzyl)-1,2,3,3a,4,9b-hexahydrochroméno[3,4-*c*]pyrrole, ses énantiomères et ses sels d'addition à un acide pharmaceutiquement acceptable.

14. Composé de formule (I) selon la revendication 1 qui est le (3aα,9bβ)-2-(4-acétylaminobenzyl)-8-cyano-1,2,3,3a,4,9b-hexahydrochroméno[3,4-*c*]pyrrole, ses énantiomères et ses sels d'addition à un acide pharmaceutiquement acceptable.

15. Procédé de préparation des composés de formule (I), caractérisé en ce que l'on utilise comme produit de départ un composé de formule (II) : 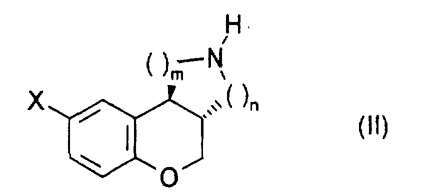 dans laquelle X, m et n ont la même signification que dans la formule (I),
que l'on traite,
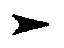 soit par un composé de formule (III) : 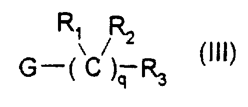 dans laquelle G représente un atome d'halogène ou un groupement CHO, q est un entier tel que 0 ≤ q ≤ 6, et R₁, R₂ et R₃ sont tels que définis dans la formule (I),
pour conduire, après réduction lorsque G représente un groupement CHO, à un dérivé de formule (l/a): 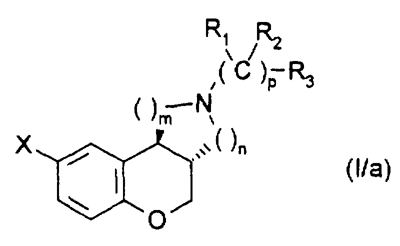 cas particulier des dérivés de formule (I) pour lesquels X, R₁, R₂, R₃, m, n et p ont la même signification que dans la formule (I),
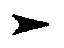 soit par un composé de formule (IV) : 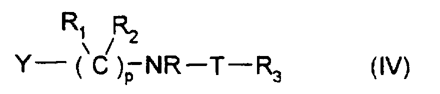 dans laquelle Y représente un atome d'halogène, R, R₁, R₂, R₃ et p sont tels que définis dans la formule (I), et T représente un groupement CO ou SO₂,
pour conduire à un dérivé de formule (l/b) : 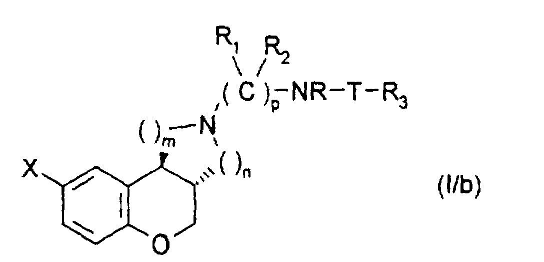 cas particulier des dérivés de formule (I), pour lesquels X, R, R₁, R₂, R₃, m, n et p ont la même signification que dans la formule (I), et T est tel que défini précédemment,
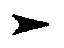 soit par un composé de formule (V) : 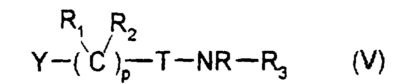 dans laquelle Y représente un atome d'halogène et R, R₁, R₂, R₃ et p sont tels que définis dans la formule (I), et T représente un groupement CO ou SO₂,
pour conduire à un dérivé de formule (l/c) : 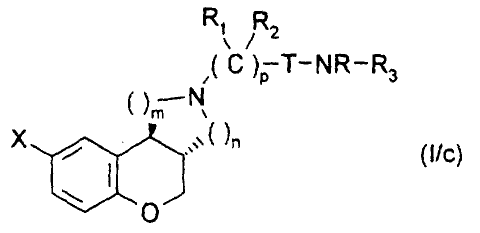 cas particulier des dérivés de formule (I), pour lequel X, R, R₁, R₂, R₃, m, n et p ont la même signification que dans la formule (I), et T est tel que défini précédemment,
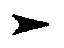 soit par un composé de formule (III') : 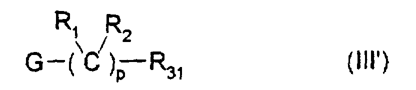 dans laquelle G représente un atome d'halogène ou un groupement CHO, p, R₁, R₂, sont tels que définis dans la formule (I), et R₃₁ représente un groupement aryle ou hétéroaryle substitué par un atome d'halogène ou groupement carboxy, nitro ou sulfo,
pour conduire à un dérivé de formule (l'/a) : 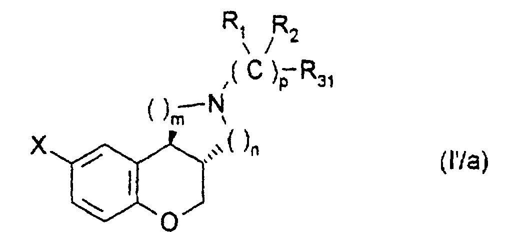 cas particulier des dérivés de formule (I/a), pour lesquels X, R₁, R₂, R₃₁, m, n et p sont tels que définis précédemment,
que l'on traite :
∗ lorsque R₃₁ représente un groupement aryle ou hétéroaryle substitué par un atome d'halogène, par un dérivé vinylique, un dérivé de l'étain ou un dérivé de l'acide boronique approprié, en présence d'un catalyseur palladié,
pour conduire au composé de formule (l/d) : 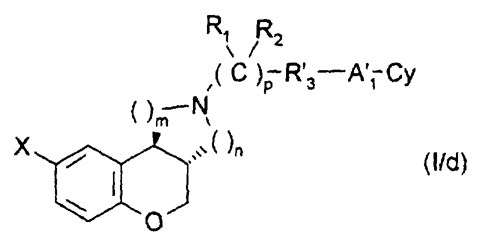 cas particulier des dérivés de formule (I), pour lequel X, R₁, R₂, R₃₁, m, n et p sont tels que définis précédemment, R'₃ représente un groupement aryle ou hétéroaryle, A'₁ représente une liaison σ ou un groupement alkylène (dans lequel un atome de carbone peut éventuellement être remplacé par un atome d'oxygène ou de soufre), alkénylène (dans lequel un atome de carbone peut éventuellement être remplacé par un atome d'oxygène ou de soufre), et Cy est tel que défini dans la formule (I),
∗ lorsque R₃₁ représente un groupement aryle ou hétéroaryle substitué par un groupement nitro, après réduction de ce groupement en amine, par un dérivé de formule CI-CO-Cy ou Cl-SO₂-Cy, pour conduire au composé de formule (l/e) : 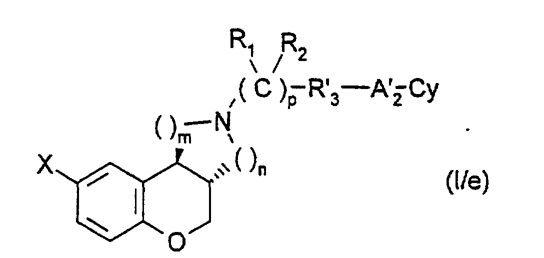 cas particulier des dérivés de formule (I), pour lequel X, R₁, R₂, m, n, p et Cy sont tels que définis dans la formule (I), R'₃ représente un groupement aryle ou hétéroaryle, et A'₂ représente un groupement -NR-CO ou -NR-SO₂-, R étant tel que défini dans la formule (I),
∗ lorsque R₃₁ représente un groupement aryle ou hétéroaryle substitué par un groupement sulfo ou carboxy, par un dérivé de formule HNR-Cy, pour conduire au composé de formule (l/f) : 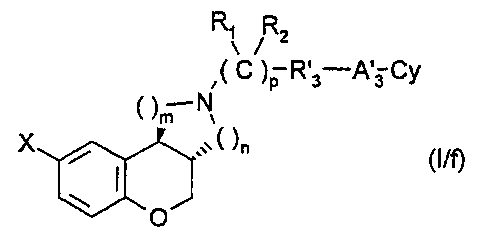 cas particulier des composés de formule (I), pour lequel X, R₁, R₂, m, n, p et Cy sont tels que définis dans la formule (I), R'₃ représente un groupement aryle ou hétéroaryle, et A'₃ représente un groupement -CO-NR-, ou -SO₂-NR-, R étant tel que défini précédemment,
étant entendu que dans les formules précédentes ( ) représente un groupement -CH₂-,
composés de formule (I/a) à (l/f) :
- que l'on purifie éventuellement selon une technique classique de purification,
- dont on sépare éventuellement les énantiomères selon une technique classique de séparation,
- et que l'on transforme, le cas échéant, en ses sels d'addition à un acide ou à une base pharmaceutiquement acceptable,
- étant entendu que lorsque X représente un groupement CN, il peut être transformé en groupement aminoalkyle selon des techniques classiques de la chimie organique, à tout moment de la synthèse.

16. Procédé de préparation des composés de formule (I) caractérisé en ce que l'on utilise comme matière première le dérivé de formule (VI) : 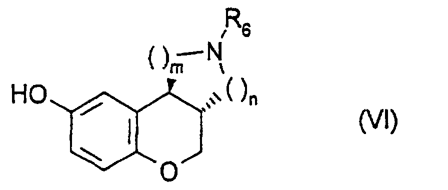 dans laquelle m et n ont la même signification que dans la formule (I) et R₆ représente un groupement alkyle (C₁-C₆) linéaire ou ramifié,
qui est traité par l'anhydride trifluorométhane sulfonique, pour conduire à un dérivé de formule (VII) : 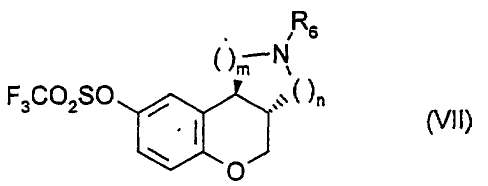 dans laquelle R₆, m et n ont la même signification que précédemment,
composé de formule (VII) qui réagit avec le cyanure de tributylétain, en présence de chlorure de lithium et d'un catalyseur au palladium (O), pour conduire au dérivé de formule (l/g) : 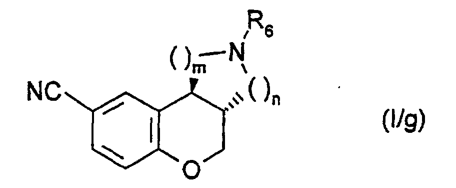 cas particulier des dérivés de formule (I) pour lesquels R₆, m et n sont tels que définis précédemment,
étant entendu que dans les formules précédentes ( ) représente un groupement -CH₂-,
composé de formule (I/g) qui peut, si nécessaire, être purifié par des méthodes classiques de purification et dont on sépare éventuellement les énantiomères par une technique classique de séparation et qui peut être transformé, le cas échéant, en son sel d'addition à un acide ou à une base pharmaceutiquement acceptable.

17. Utilisation, pour l'obtention de compositions pharmaceutiques utiles dans le traitement des maladies nécéssitant un ligand aux récepteurs D₃, des composés de formule (VIII) : 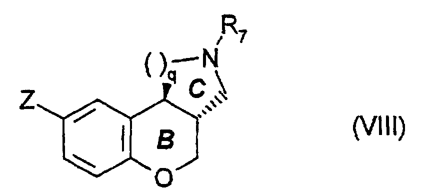 dans laquelle :
• ( ) représente un groupement -CH₂-,
• q représente 1 ou 2,
• Z représente un groupement cyano ou aminocarbonyle,
• R₇ représente un groupement alkyle (C₁-C₆) linéaire ou ramifié, benzyle, acylaminoalkyle (C₁-C₆) linéaire ou ramifié dans lequel le groupement acyle est un groupement benzoyle, naphtylcarbonyle, thienylcarbonyle, furylcarbonyle, pyrrolylcarbonyle, pyridinylcarbonyle, chacun de ces groupements étant éventuellement substitués par un ou plusieurs atomes d'halogène, ou groupements trihalogénométhyle, alkoxy ou hydroxy,
• la jonction entre les cycles B et C est de configuration trans.
ainsi que leurs énantiomères, diastéroisomères et leurs sels d'addition à un acide ou à une base pharmaceutiquement acceptable.

18. Utilisation, selon la revendication 17, pour l'obtention de compositions pharmaceutiques utiles dans le traitement des maladies nécessitant un ligand aux récepteurs D₃, d'un composé de formule (VIII) qui est le (3aα,9bβ)-2-benzyl-8-cyano-1,2,3,3a,4,9b-hexahydrochroméno[3,4-c]pyrrole, ses énantiomères et ses sels d'addition à un acide pharmaceutiquement acceptable.

19. Utilisation, selon la revendication 17, pour l'obtention de compositions pharmaceutiques utiles dans le traitement des maladies nécessitant un ligand aux récepteurs D₃, d'un composé de formule (VIII) qui est le (3aα,9bβ)-2-propyl-1,2,3,3a,4,9b-hexahydrochroméno[3,4-c]pyrrole, ses énantiomères et ses sels d'addition à un acide pharmaceutiquement acceptable.

20. Compositions pharmaceutiques contenant comme principe actif au moins un composé selon l'une quelconque des revendications 1 à 14, seul ou en combinaison avec un ou plusieurs excipients ou véhicules inertes, non toxiques, pharmaceutiquement acceptables.

21. Compositions pharmaceutiques selon la revendication 20 contenant au moins un principe actif selon l'une quelconque des revendications 1 à 14 utiles dans le traitement des maladies nécessitant un ligand aux récepteurs D₃ comme la dépression, la schizophrénie, les psychoses, la maladie de Parkinson, les troubles de la mémoire, et les troubles liés à l'abus de la drogue.

## Patentansprüche

1. Verbindungen der Formel (I): 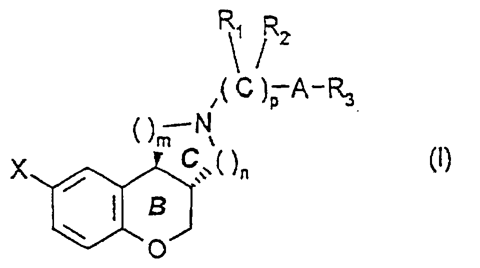 in der:
• ( ) eine Gruppe -CH₂-,
• m eine ganze Zahl von 0 ≤ m ≤ 3,
• n eine ganze Zahl von 0 ≤ n ≤ 3 und 2 ≤ m + n ≤ 3,
• p eine ganze Zahl von 1 ≤ p ≤ 6,
• die Bindung zwischen den Ringen B und C in der trans-Konfiguration vorliegt,
• X eine Cyanogruppe oder eine Gruppe -CO-NR₄R₅, worin R₄ und R₅ aus Wasserstoff, geradkettigem oder verzweigtem (C₁-C₆)-Alkyl, (C₃-C₇)-Cycloalkyl und gegebenenfalls substituiertem Aryl ausgewählt sind,
• A eine σ-Bindung oder eine Gruppe ausgewählt aus -NR-CO-, -CO-NR-, -NR-SO₂-, -SO₂-NR, worin R ein Wasserstoffatom oder eine geradkettige oder verzweigte (C₁-C₆)-Alkylgruppe darstellt,
• R₁ und R₂ unabhängig voneinander Wasserstoffatome oder geradkettige oder verzweigte (C₁-C₆)-Alkylgruppen,
• R₃:
- ein Wasserstoffatom, eine Phenyl-, Naphthyl- oder Heteroarylgruppe, wobei jede dieser Gruppen gegebenenfalls durch ein oder mehrere Halogenatome, geradkettige oder verzweigte (C₁-C₆)-Alkylgruppen, geradkettige oder verzweigte (C₁-C₆)-Alkoxygruppen, Hydroxygruppen, Cyanogruppen, Aminogruppen, Nitrogruppen, Carboxygruppen, geradkettige oder verzweigte (C₁-C₆)-Perhalogenalkylgruppen, Sulfonylgruppen, Acylaminogruppen, geradkettige oder verzweigte (C₁-C₆)-Alkylsulfonylgruppen, geradkettige oder verzweigte (C₁-C₆)-Alkylsulfonaminogruppen substituiert sind,
- eine Aryl- oder Heteroarylgruppe, die durch eine Gruppe A'-Cy substituiert ist, worin A' eine σ-Bindung oder eine geradkettige oder verzweigte (C₁-C₆)-Alkylengruppe (in der ein Kohlenstoffatom gegebenenfalls durch ein Sauerstoff- oder Schwefelatom ersetzt sein kann), eine geradkettige oder verzweigte (C₁-C₆)-Alkenylengruppe (in der ein Kohlenstoffatom gegebenenfalls durch ein Sauerstoff- oder Schwefelatom ersetzt sein kann), eine Gruppe -NR-CO-, -CO-NR, -NR-SO₂-, SO₂-NR, worin R ein Wasserstoffatom oder eine geradkettige oder verzweigte (C₁-C₆)-Alkylgruppe darstellt und Cy eine gegebenenfalls substituierte Arylgruppe oder gegebenenfalls substituierte Heteroarylgruppe darstellt,
- eine 2-Indolinon-5-yl-gruppe,
- oder eine Aryloxy- oder Arylthiogruppe, mit der Maßgabe, daß in diesem Fall A eine σ-Bindung darstellt, bedeuten,
wobei es sich versteht:
• wenn n 0 ist, m von 2 verschieden ist,
• wenn n 1, R₁ und R₂ Wasserstoff, A eine σ-Bindung und p 1 bedeuten, R₃ von Phenyl und Pyridyl verschieden ist,
• wenn n 1, R₁ und R₂ Wasserstoff und A eine σ-Bindung bedeuten, R₃ von einem Wasserstoffatom verschieden ist,
• wenn n 1, R₁ und R₂ Wasserstoff und A eine Gruppe -NH-CO- darstellen, R₃ von einem Wasserstoffatom, einer Phenylgruppe, Naphthylgruppe, einer aus Thienyl, Furyl, Pyrrolyl und Pyridyl ausgewählten heterocyclischen Gruppe, wobei jede dieser Gruppen gegebenenfalls durch ein oder mehrere Halogenatome oder Trihalogenmethylgruppen, Alkoxygruppen oder Hydroxygruppen substituiert sein können, verschieden ist,
deren Enantiomere, Diastereoisomere sowie deren Additionssalze mit einer pharmazeutisch annehmbaren Säure oder Base.

2. Verbindungen der Formel (I) nach Anspruch 1, worin m und n jeweils 1 bedeuten, deren Enantiomere, Diastereoisomere sowie deren Additionssalze mit einer pharmazeutisch annehmbaren Säure oder Base.

3. Verbindungen der Formel (I) nach Anspruch 1, worin m 3 und n 0 bedeuten, deren Enantiomere, Diastereoisomere sowie deren Additionssalze mit einer pharmazeutisch annehmbaren Säure oder Base.

4. Verbindungen der Formel (I) nach Anspruch 1, worin X eine Cyanogruppe darstellt, deren Enantiomere, Diastereoisomere sowie deren Additionssalze mit einer pharmazeutisch annehmbaren Säure oder Base.

5. Verbindungen der Formel (I) nach Anspruch 1, worin A eine σ-Bindung darstellt, deren Enantiomere, Diastereoisomere sowie deren Additionssalze mit einer pharmazeutisch annehmbaren Säure oder Base.

6. Verbindungen der Formel (I) nach Anspruch 1, worin A eine Gruppe -NR-CO- oder NR-SO₂ darstellt, deren Enantiomere, Diastereoisomere sowie deren Additionssalze mit einer pharmazeutisch annehmbaren Säure oder Base.

7. Verbindungen der Formel (I) nach Anspruch 1, worin R₃ eine gegebenenfalls substituierte Phenylgruppe oder gegebenenfalls substituierte Biphenylgruppe darstellt, deren Enantiomere, Diastereoisomere sowie deren Additionssalze mit einer pharmazeutisch annehmbaren Säure oder Base.

8. Verbindunge der Formel (I) nach Anspruch 1, worin R₃ eine durch eine Gruppe A'-Cy substituierte Arylgruppe, worin A' eine Gruppe -NR-CO oder -NR-SO₂- darstellt, bedeutet, deren Enantiomere, Diastereoisomere sowie deren Additionssalze mit einer pharmazeutisch annehmbaren Säure oder Base.

9. Verbindungen der Formel (I) nach Anspruch 1, worin X eine Cyanogruppe darstellt, m und n jeweils 1 bedeuten, R₁ und R₂ jeweils ein Wasserstoffatom darstellen und p 4 bedeutet, während A eine Gruppe NHCO und R₃ eine gegebenenfalls substituierte Phenylgruppe oder gegebenenfalls substituierte Biphenylgruppe darstellen, oder p 1 oder 2 bedeutet, während A eine σ-Bindung und R₃ eine gegebenenfalls substituierte Phenylgruppe oder gegebenenfalls substituierte Biphenylgruppe darstellt, deren Enantiomere, Diastereoisomere sowie deren Additionssalze mit einer pharmazeutisch annehmbaren Säure oder Base.

10. Verbindung der Formel (I) nach Anspruch 1, nämlich (3aα,9bβ)-2-[2-(4-Acetylaminophenyl)-ethyl)-8-cyano-1,2,3,3a,4,9b-hexahydrochromeno[3,4-*c*]pyrrol, dessen Enantiomere sowie dessen Additionssalze mit einer pharmazeutisch annehmbaren Säure.

11. Verbindung der Formel (I) nach Anspruch 1, nämlich N-[4-((3aα,9bβ)-8-Cyano- 1,2,3,3a,4,9b-hexahydrochromeno[3,4-*c*]pyrrol-2-yl)-butyl]-4-phenylbenzamid, dessen Enantiomere sowie dessen Additionssalze mit einer pharmazeutisch annehmbaren Säure.

12. Verbindung der Formel (I) nach Anspruch 1, nämlich (3aα,9bβ)-8-Cyano-2-[2-(2-oxo-2,3-dihydro-1*H*-indol-5-yl)-ethyl]-1,2,3,3a,4,9b-hexahydrochromeno[3,4-*c*]pyrrol, dessen Enantiomere sowie dessen Additionssalze mit einer pharmazeutisch annehmbaren Säure.

13. Verbindung der Formel (I) nach Anspruch 1, nämlich (3aα,9bβ)-8-Cyano-2-(4-fluorbenzyl)-1,2,3,3a,4,9b-hexahydrochromeno[3,4-c]pyrrol, dessen Enantiomere sowie dessen Additionssalze mit einer pharmazeutisch annehmbaren Säure.

14. Verbindung der Formel (I) nach Anspruch 1, nämlich (3aα,9bβ)-2-(4-Acetylaminobenzyl)-8-cyano-1,2,3,3a,4,9b-hexahydrochromeno[3,4-c]pyrrol, dessen Enantiomere sowie dessen Additionssalze mit einer pharmazeutisch annehmbaren Säure.

15. Verfahren zur Herstellung der Verbindungen der Formel (I), dadurch gekennzeichnet, daß man als Ausgangsprodukt eine Verbindung der Formel (II) verwendet: 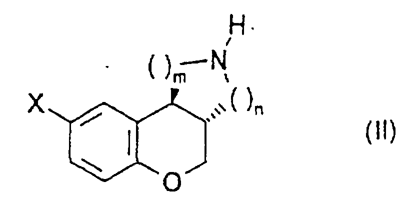 in der X, m und n die bezüglich der Formel (I) angegebenen Bedeutungen besitzen,
welche man
→ entweder mit einer Verbindung der Formel (III) umsetzt: 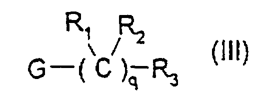 in der G ein Halogenatom oder eine CHO-Gruppe und q eine ganze Zahl von 0 ≤ q ≤ 6 bedeuten und R₁, R₂ und R₃ die bezüglich der Formel (I) angegebenen Bedeutungen besitzen,
so daß man nach der Reduktion, wenn G eine CHO-Gruppe darstellt, ein Derivat der Formel (I/a) erhält: 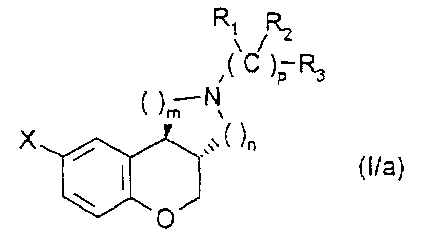 einem Sonderfall der Derivate der Formel (I), worin X, R₁,. R₂, R₃, m, n und p die bezüglich der Formel (I) angegebenen Bedeutungen besitzen,
→ oder mit einer Verbindung der Formel (IV) behandelt: 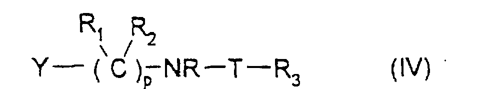 in der Y ein Halogenatom darstellt, R, R₁, R₂, R₃ und p die bezüglich der Formel (I) angegebenen Bedeutungen besitzen und T eine Gruppe CO oder SO₂ darstellt,
so daß man ein Derivat der Formel (I/b) erhält: 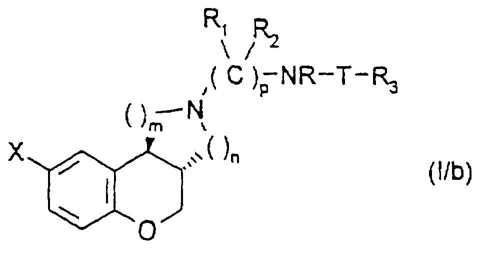 einem Sonderfall der Derivate der Formel (I), worin X, R, R₁, R₂, R₃, m, n und p die bezüglich der Formel (I) angegebenen Bedeutungen besitzen und T die oben angegebene Bedeutung besitzt,
→ oder mit einer Verbindung der Formel (V) behandelt: 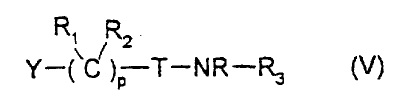 in der Y ein Halogenatom darstellt, R, R₁, R₂, R₃ und p die bezüglich der Formel (I) angegebenen Bedeutungen besitzen und T eine Gruppe CO oder SO₂ darstellt,
so daß man ein Derivat der Formel (I/c) erhält: 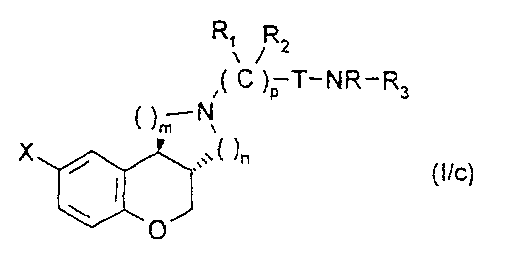 einem Sonderfall der Derivate der Formel (I), worin X, R, R₁, R₂, R₃, m, n und p die bezüglich der Formel (I) angegebenen Bedeutungen besitzen und T die oben angegebene Bedeutung besitzt,
→ oder mit einer Verbindung der Formel (III') behandelt: 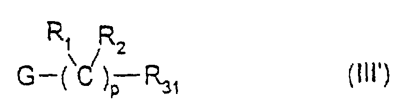 in der G ein Halogenatom oder eine Gruppe CHO darstellt, p, R₁ und R₂ die bezüglich der Formel (I) angegebenen Bedeutungen besitzen und R₃₁ eine Aryl- oder Heteroarylgruppe darstellt, die durch ein Halogenatom oder eine Carboxy-, Nitro- oder Sulfogruppe substituiert ist,
so daß man ein Derivat der Formel (I'/a) erhält: 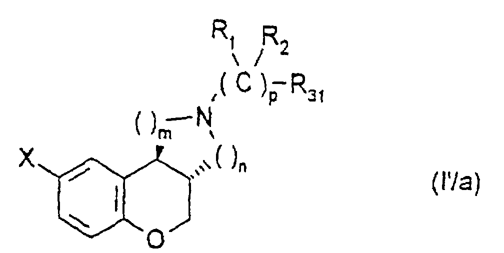 einem Sonderfall der Derivate der Formel (I/a), worin X, R₁, R₂, R₃₁, m, n und p die oben angegebenen Bedeutungen besitzen,
welches man:
* wenn R₃₁ eine Aryl- oder Heteroarylgruppe, die durch ein Halogenatom substituiert ist, darstellt, in Gegenwart eines Palladium-Katalysators mit einem geeigneten Vinylderivat, Zinnderivat oder Boronsäurederivat umsetzt,
zur Bildung der Verbindung der Formel (I/d): 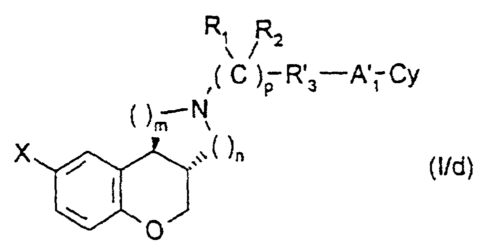 einem Sonderfall der Derivate der Formel (I), worin X, R₁, R₂, R₃₁, m, n und p die oben angegebenen Bedeutungen besitzen, R'₃ eine Aryl- oder Heteroarylgruppe und A'₁ eine σ-Bindung oder eine Alkylengruppe (in der ein Kohlenstoffatom gegebenenfalls durch ein Sauerstoff- oder Schwefelatom ersetzt sein kann), eine Alkenylengruppe (in der ein Kohlenstoffatom gegebenenfalls durch ein Sauerstoff- oder Schwefelatom ersetzt sein kann) bedeuten und Cy die bezüglich der Formel (I) angegebenen Bedeutungen besitzt,
* wenn R₃₁ eine Aryl- oder Heteroarylgruppe, die durch eine Nitrogruppe substituiert ist, nach der Reduktion dieser Gruppe zu einer Aminogruppe mit einem Derivat der Formel Cl-CO-Cy oder Cl-SO₂-Cy umsetzt zur Bildung einer Verbindung der Formel (I/e): 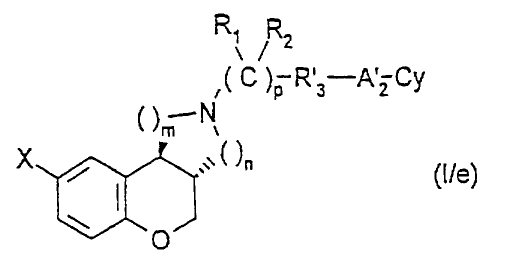 einem Sonderfall der Derivate der Formel (I), worin X, R₁, R₂, m, n, p und Cy die bezüglich der Formel (I) angegebenen Bedeutungen besitzen, R'₃ eine Aryl- oder Heteroarylgruppe darstellt und A'₂ eine Gruppe -NR-CO oder -NR-SO₂- darstellt, während R die bezüglich der Formel (I) angegebenen Bedeutungen besitzt,
* wenn R₃₁ eine Aryl- oder Heteroarylgruppe, die durch eine Sulfo- oder Carboxygruppe substituiert ist, darstellt, mit einem Derivat der Formel HNR-Cy behandelt zur Bildung der Verbindung der Formel (I/f): 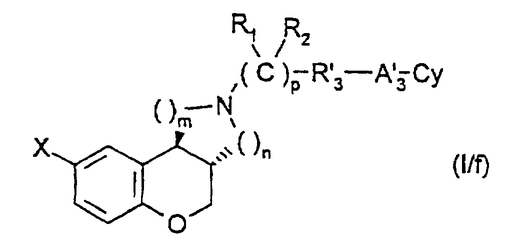 einem Sonderfall der Verbindungen der Formel (I), worin X, R₁, R₂, m, n, p und Cy die bezüglich der Formel (I) angegebenen Bedeutungen besitzen, R'₃ eine Aryl- oder Heteroarylgruppe darstellt und A'₃ eine Gruppe -CO-NR- oder -SO₂-NR- bedeutet, während R die oben angegebenen Bedeutungen besitzt,
wobei es sich versteht, daß in den obigen Formeln das Symbol ( ) für eine Gruppe -CH₂- steht,
welche Verbindungen der Formeln (I/a) bis (I/f) man:
- gegebenenfalls mit Hilfe einer klassischen Reinigungsmethode reinigt,
- gegebenenfalls mit Hilfe einer klassischen Trennmethode in ihre Enantiomeren auftrennt,
- und gegebenenfalls in ihre Additionssalze mit einer pharmazeutisch annehmbaren Säure oder Base umwandelt,
- wobei es sich versteht, daß, wenn X eine Gruppe CN darstellt, diese mit Hilfe klassischen Methoden der organischen Chemie in jedem Zeitpunkt der Synthese in eine Aminoalkylgruppe umgewandelt werden kann.

16. Verfahren zur Herstellung der Verbindungen der Formel (I), dadurch gekennzeichnet, daß man als Ausgangsmaterial das Derivat der Formel (VI) verwendet: 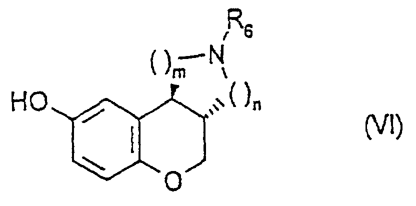 in der m und n die bezüglich der Formel (I) angegebenen Bedeutungen besitzen und R₆ eine geradkettige oder verzweigte (C₁-C₆)-Alkylgruppe darstellt,
welches mit Trifluormethansulfonsäureanhydrid behandelt wird zur Bildung eines Derivats der Formel (VII): 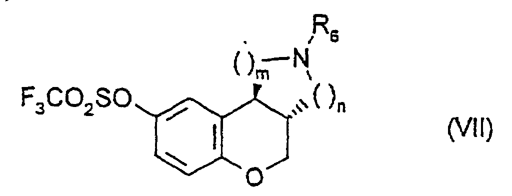 in der R₆, m und n die oben angegebenen Bedeutungen besitzen,
welche Verbindung der Formel (VII) mit Tributylzinncyanid in Gegenwart von Lithiumchlorid und einem Palladium-(0)-Katalysator umgesetzt wird zur Bildung des Derivats der Formel (I/g): 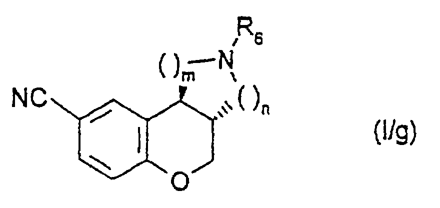 einem Sonderfall der Derivate der Formel (I), worin R₆, m und n die oben angegebenen Bedeutungen besitzen,
wobei es sich versteht, daß in den obigen Formeln das Symbol ( ) für eine Gruppe -CH₂- steht,
welche Verbindung der Formel (I/g) erforderlichenfalls mit Hilfe klassischer Reinigungsmethoden gereinigt und gegebenenfalls mit Hilfe einer klassischen Trennmethode in ihre Enantiomeren aufgetrennt und gegebenenfalls in sein Additionssalz mit einer pharmazeutisch annehmbaren Säure oder Base umgewandelt wird.

17. Verwendung der Verbindungen der Formel (VIII): 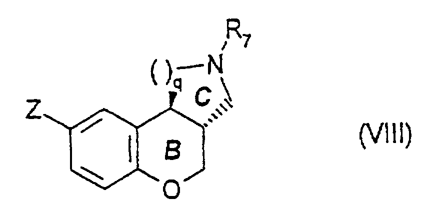 in der:
• ( ) für eine Gruppe -CH2- steht,
• q 1 oder 2 bedeutet,
• Z eine Cyano- oder Aminocarbonylgruppe bedeutet,
• R₇ eine geradkettige oder verzweigte (C₁-C₆)-Alkylgruppe, Benzylgruppe, geradkettige oder verzweigte Acylamino-(C₁-C₆)-alkylgruppe, worin die Acylgruppe eine Benzoyl-, Naphthylcarbonyl-, Thienylcarbonyl-, Furylcarbonyl-, Pyrrolylcarbonyl- oder Pyridylcarbonylgruppe, wobei jede dieser Gruppen gegebenenfalls durch ein oder mehrere Halogenatome oder Trihalogenmethylgruppen, Alkoxygruppen oder Hydroxygruppen substituiert sein kann, darstellt, bedeutet, und
• die Bindung zwischen den Ringen B und C in der trans-Konfiguration vorliegt, sowie von deren Enantiomeren, Diastereoisomeren und deren Additionssalzen mit einer pharmazeutisch annehmaren Säure oder Base zur Herstellung von pharmazeutischen Zubereitungen, die zur Behandlung von Krankheiten geeignet sind, die einen Liganden für die Rezeptoren D₃ erforderlich machen.

18. Verwendung nach Anspruch 17 einer Verbindung der Formel (VIII), nämlich von (3aα,9bβ)-2-Benzyl-8-cyano-1,2,3,3a,4,9b-hexahydrochromeno[3,4-c]pyrrol, von seinen Enantiomeren und seinen Additionssalzen mit einer pharmazeutisch annehmbaren Säure für die Herstellung von pharmazeutischen Zubereitungen, die zur Behandlung von Krankheiten geeignet sind, die einen Liganden für die Rezeptoren D₃ erforderlich machen.

19. Verwendung nach Anspruch 17 einer Verbindung der Formel (VIII), nämlich von (3aα,9bβ)-2-Propyl-1,2,3,3a,4,9b-hexahydrochromeno[3,4-c]pyrrol, von seinen Enantiomeren und seinen Additionssalzen mit einer pharmazeutisch annehmbaren Säure für die Herstellung von pharmazeutischen Zubereitungen, die zur Behandlung von Krankheiten geeignet sind, die einen Liganden für die Rezeptoren D₃ erforderlich machen.

20. Pharmazeutische Zubereitungen enthaltend als Wirkstoff mindestens eine Verbindung nach einem der Ansprüche 1 bis 14 allein oder in Kombination mit einem oder mehreren inerten, nichttoxischen, pharmazeutisch annehmbaren Trägermaterialien oder Bindemitteln.

21. Pharmazeutische Zubereitungen nach Anspruch 20 enthaltend mindestens einen Wirkstoff nach einem der Ansprüche 1 bis 14 zur Behandlung von Erkrankungen, die einen Liganden für die Rezeptoren D₃ erforderlich machen, wie von Depressionen, der Schizophrenie, von Psychosen, der Parkinsonschen Krankheit, von Gedächtnisstörungen und Störungen, die mit dem Drogenmißbrauch verknüpft sind.

## Claims

1. Compounds of formula (I): 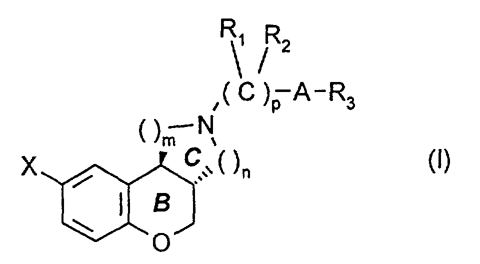 wherein :
• ( ) represents a -CH₂- group,
• m is an integer such that 0 ≤ m ≤ 3,
• n in an integer such that 0 ≤ n ≤ 3 and 2 ≤ m+n ≤ 3,
• p is an integer such that 1 ≤ p ≤ 6,
• the junction between the *B* and *C* rings is in the *trans* configuration,
• X represents a cyano group or a group -CO-NR₄R₅, R₄ and R₅ being selected from hydrogen, linear or branched (C₁-C₆)-alkyl, (C₃-C₇)-cycloalkyl and optionally substituted aryl,
• A represents a σ bond or a group selected from -NR-CO-, -CO-NR-, -NR-SO₂ and -SO₂-NR wherein R represents a hydrogen atom or a linear or branched (C₁-C₆)-alkyl group,
• R₁ and R₂ each independently represent a hydrogen atom or a linear or branched (C₁-C₆)-alkyl group,
• R₃ represents:
- a hydrogen atom, or a phenyl, naphthyl or heteroaryl group each of which is optionally substituted by one or more halogen atoms, linear or branched (C₁-C₆)-alkyl, linear or branched (C₁-C₆)-alkoxy, hydroxy, cyano, amino, nitro, carboxy, linear or branched (C₁-C₆)-perhaloalkyl, sulphonyl, acylamino, linear or branched (C₁-C₆)-alkylsulphonyl, linear or branched (C₁-C₆)-alkylsulphonylamino groups,
- an aryl or heteroaryl group substituted by a group A'-Cy wherein A' represents a σ bond, a linear or branched (C₁-C₆)-alkylene group (in which a carbon atom may optionally be replaced by an oxygen or sulphur atom), a linear or branched (C₁-C₆)-alkenylene group (in which a carbon atom may optionally be replaced by an oxygen or sulphur atom) or a group -NR-CO-, -CO-NR, -NR-SO₂- or SO₂-NR in which R represents a hydrogen atom or a linear or branched (C₁-C₆)-alkyl group, and Cy represents an optionally substituted aryl group or an optionally substituted heteroaryl group,
- a 2-indolinon-5-yl group,
- or an aryloxy or arylthio group, with the proviso that in that case A represents a σ bond,
provided that:
• when n is 0, m is other than 2,
• when n is 1, R₁ and R₂ represent a hydrogen atom, A represents a σ bond and p is 1, R₃ is other than phenyl or pyridyl,
• when n is 1, R₁ and R₂ represent a hydrogen atom and A represents a σ bond, R₃ is other than a hydrogen atom,
• when n is 1, R₁ and R₂ represent a hydrogen atom and A represents an -NH-CO- group, R₃ is other than a hydrogen atom, a phenyl group, a naphthyl group or a heterocyclic group selected from thienyl, furyl, pyrrolyl and pyridyl, each of those groups being optionally substituted by one or more halogen atoms or trihalomethyl, alkoxy or hydroxy groups,
their enantiomers, diastereoisomers, and also the addition salts thereof with a pharmaceutically acceptable acid or base.

2. Compounds of formula (I) according to claim 1 wherein m and n are each 1, their enantiomers, diastereoisomers, and also the addition salts thereof with a pharmaceutically acceptable acid or base.

3. Compounds of formula (I) according to claim 1 wherein m is 3 and n is 0, their enantiomers, diastereoisomers, and also the addition salts thereof with a pharmaceutically acceptable acid or base.

4. Compounds of formula (I) according to claim 1 wherein X represents a cyano group, their enantiomers, diastereoisomers, and also the addition salts thereof with a pharmaceutically acceptable acid or base.

5. Compounds of formula (I) according to claim 1 wherein A represents a σ bond, their enantiomers, diastereoisomers, and also the addition salts thereof with a pharmaceutically acceptable acid or base.

6. Compounds of formula (I) according to claim 1 wherein A represents an -NR-CO- or NR-SO₂ group, their enantiomers, diastereoisomers, and also the addition salts thereof with a pharmaceutically acceptable acid or base.

7. Compounds of formula (I) according to claim 1 wherein R₃ represents an optionally substituted phenyl group or an optionally substituted biphenyl group, their enantiomers, diastereoisomers, and also the addition salts thereof with a pharmaceutically acceptable acid or base.

8. Compounds of formula (I) according to claim 1 wherein R₃ represents an aryl group substituted by a group A'-Cy wherein A' represents an -NR-CO or -NR-SO₂- group, their enantiomers, diastereoisomers, and also the addition salts thereof with a pharmaceutically acceptable acid or base.

9. Compounds of formula (I) according to claim 1 wherein X represents a cyano group, m and n are each 1, R₁ and R₂ each represent a hydrogen atom, and p is 4 when A represents an NHCO group and R₃ represents an optionally substituted phenyl group or an optionally substituted biphenyl group, or p is 1 or 2 when A represents a σ bond and R₃ represents an optionally substituted phenyl group or an optionally substituted biphenyl group, their enantiomers, diastereoisomers, and also the addition salts thereof with a pharmaceutically acceptable acid or base.

10. Compound of formula (I) according to claim 1 which is (3aα,9bβ)-2-[2-(4-acetylaminophenyl)ethyl]-8-cyano-1,2,3,3a,4,9b-hexahydrochromeno[3,4-c]pyrrole, its enantiomers, and also the addition salts thereof with a pharmaceutically acceptable acid.

11. Compound of formula (I) according to claim 1 which is N-[4-((3aα,9bβ)-8-cyano-1,2,3,3a,4,9b-hexahydrochromeno[3,4-c]pyrrol-2-yl)butyl]-4-phenylbenzamide, its enantiomers, and also the addition salts thereof with a pharmaceutically acceptable acid.

12. Compound of formula (I) according to claim 1 which is (3aα,9bβ)-8-cyano-2-[2-(2-oxo-2,3-dihydro-*1H*-indol-5-yl)ethyl]-1,2,3,3a,4,9b-hexahydrochromeno[3,4-c]pyrrole, its enantiomers, and also the addition salts thereof with a pharmaceutically acceptable acid.

13. Compound of formula (I) according to claim 1 which is (3aα,9bβ)-8-cyano-2-(4-fluorobenzyl)-1,2,3,3a,4,9b-hexahydrochromeno[3,4-c]pyrrole, its enantiomers, and also the addition salts thereof with a pharmaceutically acceptable acid.

14. Compound of formula (I) according to claim 1 which is (3aα,9bβ)-2-(4-acetylaminobenzyl)-8-cyano-1,2,3,3a,4,9b-hexahydrochromeno[3,4-*c*]pyrrole, its enantiomers, and also the addition salts thereof with a pharmaceutically acceptable acid.

15. Process for the preparation of compounds of formula (I), characterised in that there is used as starting material a compound of formula (II) : 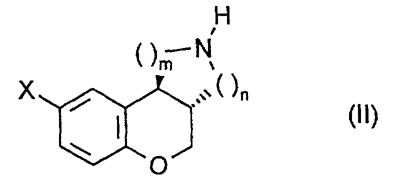 wherein X, m and n are as defined for formula (I),
which is treated
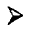 with a compound of formula (III) : 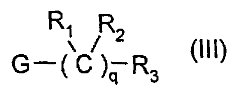 wherein G represents a halogen atom or a CHO group, q is an integer such that 0 ≤ q ≤ 6, and R₁, R₂ and R₃ are as defined for formula (I),
to yield, after reduction when G represents a CHO group, a compound of formula (l/a): 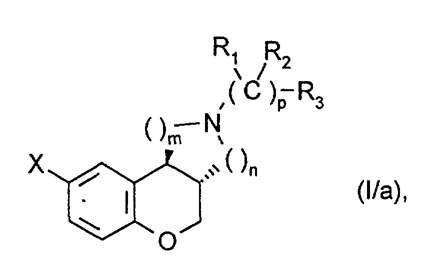 a particular case of compounds of formula (I) wherein X, R₁, R₂, R₃, m, n and p are as defined for formula (I),
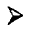 or with a compound of formula (IV) : 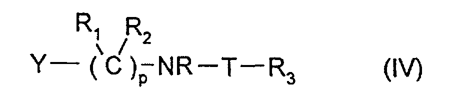 wherein Y represents a halogen atom, R, R₁, R₂, R₃ and p are as defined for formula (I) and T represents a CO or SO₂ group,
to yield a compound of formula (l/b) : 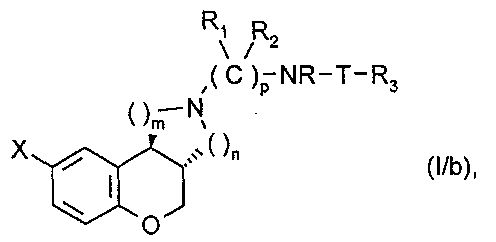 a particular case of compounds of formula (I) wherein X, R, R₁, R₂, R₃, m, n and p are as defined for formula (I) and T is as defined above,
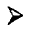 or with a compound of formula (V): 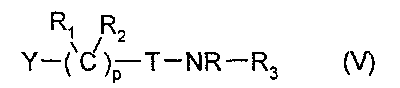 wherein Y represents a halogen atom, R, R₁, R₂, R₃ and p are as defined for formula (I) and T represents a CO or SO₂ group,
to yield a compound of formula (I/c) : 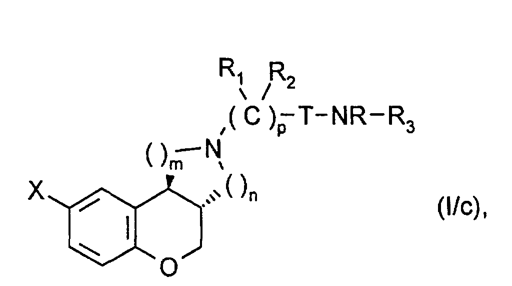 a particular case of compounds of formula (I) wherein X, R, R₁, R₂, R₃, m, n and p are as defined for formula (I) and T is as defined above,
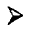 or with a compound of formula (III') : 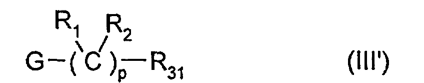 wherein G represents a halogen atom or a CHO group, p, R₁ and R₂ are as defined for formula (I) and R₃₁ represents an aryl or heteroaryl group substituted by a halogen atom or by a carboxy, nitro or sulpho group,
to yield a compound of formula (l'/a) : 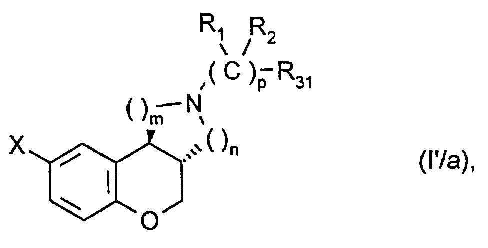 a particular case of compounds of formula (I/a) wherein X, R₁, R₂, R₃₁, m, n and p are as defined hereinbefore,
which :
* when R₃₁ represents an aryl or heteroaryl group substituted by a halogen atom, is treated with an appropriate vinyl compound, tin compound or boronic acid compound in the presence of a palladium catalyst to yield a compound of formula (I/d) : 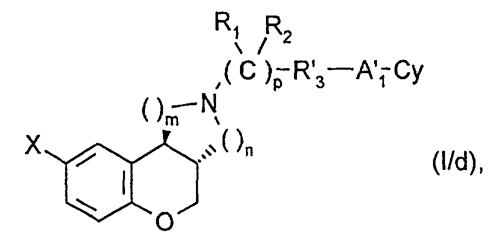 a particular case of compounds of formula (I) wherein X, R₁, R₂, R₃₁, m, n and p are as defined hereinbefore, R'₃ represents an aryl or heteroaryl group, A'₁ represents a σ bond, an alkylene group (in which a carbon atom may optionally be replaced by an oxygen or sulphur atom) or an alkenylene group (in which a carbon atom may optionally be replaced by an oxygen or sulphur atom) and Cy is as defined for formula (I),
* when R₃₁ represents an aryl or heteroaryl group substituted by a nitro group, after reduction of that group to amine, is treated with a compound of formula Cl-CO-Cy or Cl-SO₂-Cy to yield a compound of formula (l/e) : 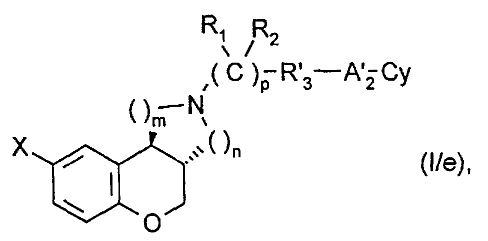 a particular case of compounds of formula (I) wherein X, R₁, R₂, m, n, p and Cy are as defined for formula (I), R'₃ represents an aryl or heteroaryl group and A'₂ represents an -NR-CO or -NR-SO₂- group, R being as defined for formula (I),
* when R₃₁ represents an aryl or heteroaryl group substituted by a sulpho or carboxy group, is treated with a compound of formula HNR-Cy to yield a compound of formula (l/f): 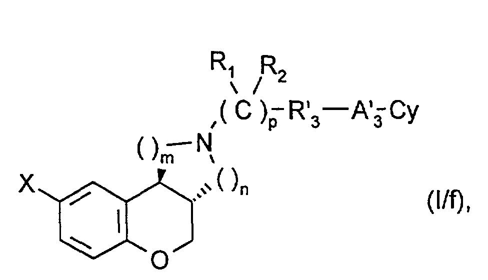 a particular case of compounds of formula (I) wherein X, R₁, R₂, m, n, p and Cy are as defined for formula (I), R'₃ represents an aryl or heteroaryl group and A'₃ represents a -CO-NR- or -SO₂-NR- group, R being as defined hereinbefore,
it being understood that in the above formula () represents a -CH₂- group,
which compounds of formulae (I/a) to (l/f):
- are purified, if necessary, according to a conventional purification technique,
- are separated, if desired, into the enantiomers according to a conventional separation technique,
- are converted, if desired, into addition salts with a pharmaceutically acceptable acid or base,
- with the proviso that when X represents a CN group it may be converted into an aminoalkyl group according to conventional techniques of organic chemistry at any stage of the synthesis.

16. Process for the preparation of compounds of formula (I), characterised in that there is used as starting material a compound of formula (VI) : 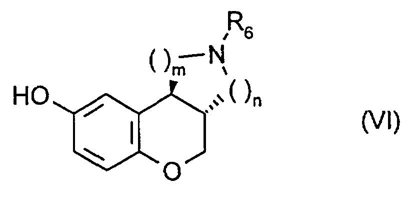 wherein m and n are as defined for formula (I) and R₆ represents a linear or branched (C₁-C₆)-alkyl group,
which is treated with trifluoromethanesulphonic anhydride to yield a compound of formula (VII) : 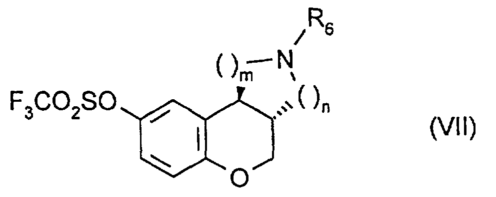 wherein R₆, m and n are as defined hereinbefore,
which compound of formula (VII) reacts with tributyltin cyanide in the presence of lithium chloride and a palladium(0) catalyst to yield a compound of formula (I/g) : 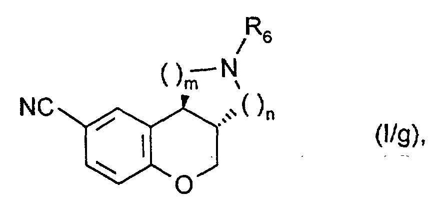 a particular case of compounds of formula (I) wherein R₆, m and n are as defined hereinbefore,
it being understood that in the above formula ( ) represents a -CH₂- group,
which compound of formula (I/g) may, if necessary, be purified by conventional purification methods and may, if desired, be separated into the enantiomers by a conventional separation technique and may, if desired, be converted into an addition salt with a pharmaceutically acceptable acid or base.

17. Use, for the manufacture of pharmaceutical compositions for use in the treatment of disorders requiring a D₃ receptor ligand, of compounds of formula (VIII) : 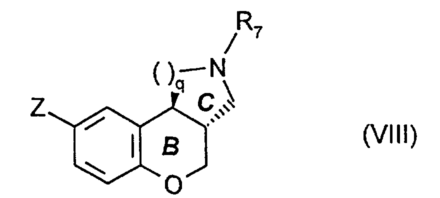 wherein :
• () represents a -CH2- group,
• q represents 1 or 2,
• Z represents a cyano or aminocarbonyl group,
• R₇ represents a linear or branched (C₁-C₆)-alkyl group, a benzyl group or an acylamino-(C₁-C₆)-alkyl group in which the alkyl moiety is linear or branched and in which the acyl group is a benzoyl, naphthylcarbonyl, thienylcarbonyl, furylcarbonyl, pyrrolylcarbonyl or pyridinylcarbonyl group each of which is optionally substituted by one or more halogen atoms or trihalomethyl, alkoxy or hydroxy groups,
• the junction between the rings B and C is in the *trans* configuration,
and also their enantiomers, diastereoisomers and the addition salts thereof with a pharmaceutically acceptable acid or base.

18. Use according to claim 17, for obtaining pharmaceutical compositions for use in the treatment of disorders requiring a D₃ receptor ligand, of a compound of formula (VIII) which is (3aα,9bβ)-2-benzyl-8-cyano-1,2,3,3a,4,9b-hexahydrochromeno[3,4-c]pyrrole, its enantiomers and addition salts thereof with a pharmaceutically acceptable acid.

19. Use according to claim 17, for obtaining pharmaceutical compositions for use in the treatment of disorders requiring a D₃ receptor ligand, of a compound of formula (VIII) which is (3aα,9bβ)-2-propyl-1,2,3,3a,4,9b-hexahydrochromeno[3,4-c]pyrrole, its enantiomers and addition salts thereof with a pharmaceutically acceptable acid.

20. Pharmaceutical compositions comprising as active ingredient at least one compound according to any one of claims 1 to 14, alone or in combination with one or more pharmaceutically acceptable, inert, non-toxic excipients or carriers.

21. Pharmaceutical compositions according to claim 20 containing at least one active ingredient according to any one of claims 1 to 14 for use in the treatment of disorders requiring a D₃ receptor ligand, such as depression, schizophrenia, psychoses, Parkinson's disease, memory disorders, and disorders associated with drug abuse.
